(19) Europäisches Patentamt
European Patent Office
Office européen des brevets

(11) EP 3 854 872 A1

(12) **EUROPEAN PATENT APPLICATION**
published in accordance with Art. 153(4) EPC

(43) Date of publication:
**28.07.2021 Bulletin 2021/30**

(21) Application number: **18936847.5**

(22) Date of filing: **11.10.2018**

(51) Int Cl.:
***C12N 9/12*** (2006.01) ***C12N 15/52*** (2006.01)
***C12N 15/63*** (2006.01)

(86) International application number:
**PCT/CN2018/109777**

(87) International publication number:
**WO 2020/073266 (16.04.2020 Gazette 2020/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BGI Shenzhen**
**Shenzhen, Guangdong 518083 (CN)**

(72) Inventors:
- **ZHANG, Zhougang**
  **Shenzhen Guangdong 518083 (CN)**
- **LIU, Huanhuan**
  **Shenzhen Guangdong 518083 (CN)**
- **ZHENG, Yue**
  **Shenzhen Guangdong 518083 (CN)**
- **ZHOU, Yujun**
  **Shenzhen Guangdong 518083 (CN)**
- **XIA, Jun**
  **Shenzhen Guangdong 518083 (CN)**
- **DONG, Yuliang**
  **Shenzhen Guangdong 518083 (CN)**
- **XU, Chongjun**
  **Shenzhen Guangdong 518083 (CN)**
- **ZHANG, Wenwei**
  **Shenzhen Guangdong 518083 (CN)**

(74) Representative: **Biggi, Cristina**
**Bugnion S.p.A.**
**Viale Lancetti 17**
**20158 Milano (IT)**

# (54) PHI29 DNA POLYMERASE MUTANT WITH IMPROVED THERMAL STABILITY AND APPLICATION THEREOF IN SEQUENCING

(57) Provided are a Phi29 DNA polymerase mutant with improved thermal stability and an use thereof in sequencing. The phi29 DNA polymerase mutant is represented by A) or B) below: the DNA polymerase mutant represented by the A) is a protein having DNA polymerase activity that is obtained by modifying at least one amino acid residue(s) in the following six sites in a phi29 DNA polymerase amino acid sequence: the 97-th, 123-th, 217-th, 224-th, 515-th, and 474-th sites; the DNA polymerase mutant represented by the B) is a protein having DNA polymerase activity that is derived from the A) by adding a tag sequence to a terminal of the amino acid sequence of the protein represented by the A).

EP 3 854 872 A1

## Description

### Technical Field

The disclosure belongs to the field of biotechnologies, and particularly relates to a Phi29 DNA polymerase mutant with improved thermal stability and an application thereof in sequencing

### Background

A Phi29 DNA polymerase, belonging to a family-B DNA polymerase, is a polymerase derived from *Bacillus subtilis* Phi29 phage. The crystal structure of the Phi29 DNA polymerase shows that, in addition to conserved Palm, Thumb, Finger and Exo domain (having 3'→5' exonuclease correction activity) of the common family-B DNA polymerase, the Phi29 DNA polymerase also has two other unique domains: TPR1 and TPR2 domains. The TPR2 domain participates in formation of a narrow channel surrounding a downstream DNA strand template, and the dissociation of a double-stranded DNA is forced; at the same time, the Palm, Thumb, TPR1 and TPR2 domains form a circular structure, and tightly bind to the double-stranded DNA formed by an upstream template strand. The structural characteristics of the Phi29 DNA polymerase endow it with a special high sustained synthesis ability, a stronger strand displacement function and a high fidelity. It is often used in applications such as constant temperature amplification such as rolling circle amplification (RCA) of a trace circular DNA or multiple-strand displacement amplification of a genome, for example, a DNA Nanoball Technology of a Beijing Genomics Institute (BGI) gene sequencer and a single-cell whole-gene sequencing technology.

The DNA nanoball technology (DNB Technology) is one of core technologies of the BGI gene sequencer. After a genomic DNA is physically or enzymatically fragmented, a linker is added and it is cyclized into a single-stranded circular DNA, and then the DNA polymerase is replaced with a high-fidelity strand, for example, the Phi29 DNA polymerase or Bst DNA polymerase performs the rolling circle amplification (RCA) on the single-stranded circular DNA, and an amplified product is called as a DNA nanoball (DNB). The nanoball is fixed on an arrayed silicon chip through a DNB loading technology for subsequent on-machine sequencing. Different properties of Phi29 DNA polymerase mutants have different binding abilities to a substrate DNA. The amplified DNA nanoball products are also different in size, uniformity, branch structure, compactness and other characteristics, and then after the DNB is loaded on the silicon chip, the quality of sequencing is also different. Properties of the Phi29 DNA polymerase also affect an amplification effect of a single-cell whole genome amplified by a Multiple Displacement Amplification (MDA) method, and the quality of single-cell sequencing is affected, for example, a Coverage, a Mapping Rate, a mutation detection accuracy and specificity, these are also very concerned parameters in application of a single-cell sequencing scientific research.

The Phi29 DNA polymerase is a medium-temperature polymerase, and may lose activity after being heated at 65°C for 10 min. Some commercial Phi29 DNA polymerases on the market, due to reasons such as stability, are difficult to meet requirements of kit product development in special applications such as sequencing library amplification and single-cell whole genome amplification because of problems in aspects such as an effective signal site on a chip, an amplification preference, a coverage, and variation detection and evaluation.

### Summary

The disclosure provides a protein.

The protein provided by the disclosure is a Phi29 DNA polymerase mutant, and is represented by A) or B) below:

> the protein represented by the A) is a protein having DNA polymerase activity that is obtained by modifying at least one amino acid residue in the following 6 sites in a phi29 DNA polymerase amino acid sequence: the 97-th, 123-th, 217-th, 224-th, 515-th, and 474-th sites, herein the rest amino acid residues are not changed; and
>
> the protein represented by the B) is a protein having DNA polymerase activity that is derived from the A) by adding a tag sequence to a terminal of the amino acid sequence of the protein represented by the A).

In the above protein, the protein represented by the A) is a protein having DNA polymerase activity that is obtained by modifying amino acid residues in at least 2 or at least 3 or at least 4 or at least 5 or all of the following 6 sites in the phi29 DNA polymerase amino acid sequence: the 97-th, 123-th, 217-th, 224-th, 515-th, and 474-th sites.

The above modification is to modify only the 6 sites of the 97-th, 123-th, 217-th 224-th, 515-th and 474-th sites in the phi29 DNA polymerase amino acid sequence according to the above sites to be modified, and the rest amino acid residues are not changed except the 6 sites.

In the above protein, the protein represented by the A) is a protein having DNA polymerase activity that is obtained by modifying amino acid residues in all of the following 5 sites in the phi29 DNA polymerase amino acid sequence: the 97-th, 123-th, 217-th, 224-th, and 515-th sites, herein the rest amino acid residues are not changed. Specifically, it is phi29-337 in an embodiment.

In the above protein, the protein represented by the A) is a protein having DNA polymerase activity that is obtained by modifying amino acid residues in all of the following 3 sites in the phi29 DNA polymerase amino acid sequence: the 224-th, 515-th and 474-th sites, herein the rest amino acid residues are not changed. Specifically, it is phi29-464 in an embodiment.

In the above protein, the protein represented by the A) is a protein having DNA polymerase activity that is obtained by modifying amino acid residues in all of the following 3 sites in the phi29 DNA polymerase amino acid sequence: the 97-th, 123-th and 515-th sites, herein the rest amino acid residues are not changed. Specifically, it is phi29-414 in an embodiment.

In the above protein, the protein represented by the A) is a protein having DNA polymerase activity that is obtained by modifying amino acid residues in all of the following 4 sites in the phi29 DNA polymerase amino acid sequence: the 97-th, 123-th, 217-th and 515-th sites, herein the rest amino acid residues are not changed. Specifically, it is phi29-458 in an embodiment.

In the above protein, the protein represented by the A) is a protein having DNA polymerase activity that is obtained by modifying amino acid residues in the following 4 sites in the phi29 DNA polymerase amino acid sequence: the 97-th, 123-th, 224-th, and 515-th sites, herein the rest amino acid residues are not changed. Specifically, it is phi29-459 in an embodiment.

In the above protein, the protein represented by the A) is a protein having DNA polymerase activity that is obtained by modifying amino acid residues in the following 2 sites in the phi29 DNA polymerase amino acid sequence: the 224-th and 474-th sites, herein the rest amino acid residues are not changed. Specifically, it is phi29-463 in an embodiment.

In the above protein, the protein represented by the A) is a protein having DNA polymerase activity that is obtained by modifying amino acid residues in the following 2 sites in the phi29 DNA polymerase amino acid sequence: the 217-th and 224-th sites, herein the rest amino acid residues are not changed. Specifically, it is phi29-335 in an embodiment.

In the above protein, the modification is amino acid substitution.

In the above protein, the amino acid substitutions in 6 sites of the 97-th, 123-th, 217-th, 224-th, 515-th and 474-th sites are respectively as follows:

a methionine in the 97-th site is substituted with an alanine or a histidine or a lysine or a threonine;

a leucine in the 123-th site is substituted with a lysine or a phenylalanine or an isoleucine or a histidine;

a glycine in the 217-th site is substituted with a glutamic acid;

a tyrosine in the 224-th site is substituted with a lysine;

an isoleucine in the 474-th site is substituted with a lysine; and

a glutamic acid in the 515-th site is substituted with a proline or a glycine.

In the above protein, the protein represented by the A) is a protein having DNA polymerase activity that is obtained by mutating the amino acid residue from M into K in the 97-th site, from L into H in the 123-th site, from E into P in the 515-th site, from Y into K in the 224-th site, and from G into K in the 217-th site of the phi29 DNA polymerase amino acid sequence, herein the rest amino acid residues are not changed.

In the above protein, the protein represented by the A) is a protein that is obtained by mutating the amino acid residue from Y into K in the 224-th site, from I into K in the 474-th site, and from E into P in the 515-th site of the phi29 DNA polymerase amino acid sequence.

In the above protein, the protein represented by the A) is a protein that is obtained by mutating the amino acid residue from M into T in the 97-th site, from L into H in the 123-th site, and from E into P in the 515-th site of the phi29 DNA polymerase amino acid sequence.

In the above protein, the phi29 DNA polymerase amino acid sequence is (I) or (II) or (III) below:

(I) a protein shown in SEQ ID NO: 2 of a sequence listing;

(II) a protein having more than 90% identity with the protein shown in SEQ ID NO: 2 of the sequence listing and derived from a Bacillus subtilis protein; and

(III) a protein having more than 95% identity with the protein shown in SEQ ID NO: 2 of the sequence listing and derived from a Bacillus subtilis protein.

In the above protein, the stability of the protein is higher than that of the phi29 DNA polymerase.
In the above protein, the stability is thermal stability.
A nucleic acid molecule for encoding the above protein is also within a scope of protection of the disclosure.
An expression cassette, a recombinant vector, recombinant bacteria or a transgenic cell line containing the above nucleic acid molecule is also within a scope of protection of the disclosure.
Use of the above protein in at least one of the following 1)-11) is also within a scope of protection of the disclosure:

1) as a DNA polymerase;

2) catalyzing DNA replication and/or catalyzing DNA amplification;

3) catalyzing rolling circle amplification and/or catalyzing multiple-strand displacement amplification;

4) performing DNA sequencing or RNA sequencing or whole genome sequencing;

5) constructing a RCA library;

6) detecting a genome amplification coverage;

7) preparing a kit for catalyzing the DNA replication and/or catalyzing the DNA amplification;

8) preparing a product for catalyzing the rolling circle amplification and/or catalyzing the multiple-strand displacement amplification;

9) preparing a product for performing the DNA sequencing or the RNA sequencing or the whole genome sequencing;

10) preparing a product for constructing the RCA library; and

11) preparing a product for detecting the genome amplification coverage.

Use of the nucleic acid molecule for encoding the above protein, the expression cassette containing the nucleic acid molecule, the recombinant vector containing the nucleic acid molecule, the recombinant bacteria containing the nucleic acid molecule, or the transgenic containing the nucleic acid molecule in at least one of the following 1)-10) is also within a scope of protection of the disclosure:

1) catalyzing DNA replication and/or catalyzing DNA amplification;

2) catalyzing rolling circle amplification and/or catalyzing multiple-strand displacement amplification;

3) performing DNA sequencing or RNA sequencing or whole genome sequencing;

4) constructing a RCA library;

5) detecting a genome amplification coverage;

6) preparing a product for catalyzing the DNA replication and/or catalyzing the DNA amplification;

7) preparing a product for catalyzing the rolling circle amplification and/or catalyzing the multiple-strand displacement amplification;

8) preparing a product for performing the DNA sequencing or the RNA sequencing or the whole genome sequencing;

9) preparing a product for constructing the RCA library; and

10) preparing a product for detecting the genome amplification coverage.

Another purpose of the disclosure is to provide a method for improving stability of a phi29 DNA polymerase.
The method provided by the disclosure includes the following steps: modifying at least one amino acid residue(s) in the following 6 sites of the phi29 DNA polymerase amino acid sequence: the 97-th, 123-th, 217-th, 224-th, 515-th and 474-th sites, in the modification modes above, herein the rest amino acid residues are not changed, to obtain a protein having DNA polymerase activity.
In the above method, the stability is thermal stability.
The Phi29 DNA polymerase of the disclosure may exist in the form of an independently packaged DNA polymerase product, or may be packaged into a DNA amplification kit or a DNA sequencing kit. The disclosure is capable of, through site-directed mutagenesis and screening technologies to obtain the mutant with the greatly improved thermal stability. Herein the amplification coverage of some mutants in single-cell whole genome amplification is greatly improved, and the accuracy and sensitivity of the mutation detection are improved, and reach a same level of QIAGEN similar products; and other mutants in the disclosure have a good effect on improving a DNB loading effect in application of RCA library amplification.
The recombinant vector is obtained by inserting the nucleic acid molecule into an expression vector.
The expression vector may specifically be a pET28a(+) vector.
The recombinant bacteria are bacteria obtained by introducing the recombinant vector into original bacteria.
The original bacteria may be *Escherichia coli.*
The *E. coli* may specifically be *Escherichia coli* BL21 (DE3).
The transgenic cell line may be obtained by transforming the recombinant vector into recipient cells. The transgenic cell line is a non-plant propagative material.
The stability may specifically be thermal stability. The thermal stability may specifically be thermal stability at 37°C.
The phi29 DNA polymerase may be (I) or (II) or (III) below:

(I) a protein shown in SEQ ID NO: 2 of a sequence listing;

(II) a protein having more than 90% identity with the protein shown in SEQ ID NO: 2 of the sequence listing and derived from a Bacillus subtilis protein; and

(III) a protein having more than 95% identity with the protein shown in SEQ ID NO: 2 of the sequence listing and derived from a Bacillus subtilis protein.

## Brief Description of the Drawings

Fig. 1 is a construction schematic diagram of a Phi29 gene expression vector.

Fig. 2 is a coverage of a housekeeping gene in a MDA product detected by multiple PCR.

Fig. 3 is data analysis of an on-machine test of RCA library construction.

## Detailed Description of the Embodiments

The following embodiments are convenient for understanding the disclosure better, but do not limit the disclosure. Unless otherwise specified, experimental methods in the following embodiments are all conventional methods. Unless otherwise specified, test materials used in the following embodiments are all purchased from conventional biochemical reagent stores. Quantitative tests in the following embodiments are all set to three times of repeated experiments, and results are averaged. For each solution or buffer in the following embodiments, unless otherwise specified, solvents are all water.
pET28a(+) vector: Novagen Company.
*Escherichia coli* BL21 (DE3): TIANGEN, CB105-02.
Storage buffer: 10 mM Tris-HCI, 100 mM KCI, 1 mM DTT, 0.1 mM EDTA, 0.5% (v/v) Tween20, 0.5% (v/v) NP-40, 50% (v/v) Glycerol, and pH 7. 4@25°C.
141 RCA Primer in the following embodiments: TCCTAAGACCGCTTGGCCTCCGACT.
141 Ad ssDNA in the following embodiments: self-made by BGI, a single-stranded loop library of a certain size range, without a fixed sequence (a random library formed by four nucleotides A/T/C/G, and a main band length is 200-300 bp).

In the following embodiments, a DNA molecule shown in SEQ ID NO: 1 of a sequence listing is an encoding gene of a wild-type Phi29 DNA polymerase, and it expresses a protein shown in SEQ ID NO: 2 of the sequence listing, namely the wild-type Phi29 DNA polymerase (represented by WT).

Embodiment 1: Preparation of Phi29 DNA polymerase mutant

A Phi29 DNA polymerase mutant is obtained by mutating at least one of the 97-th, 123-th, 217-th, 224-th, 474-th, and 515-th sites in an amino acid sequence of a wild-type Phi29 DNA polymerase. Specifically, a single-site mutation form is shown in Table 1, and a multi-site combination mutation form is shown in Table 2.

I. Construction of a recombinant vector for expressing Phi29 DNA polymerase mutant

1. Recombinant vector for expressing Phi29 DNA polymerase single-site mutant

A DNA molecule shown in SEQ ID NO: 2 of a sequence listing is inserted between NdeI and BamHI restriction sites of a pET28a(+) vector (Fig. 1), to obtain a recombinant vector WT for expressing a wild-type Phi29 DNA polymerase.

The recombinant vector WT is served as an original vector, each primer pair shown in Table 1 is used to introduce a site mutation, and each recombinant vector for expressing the Phi29 DNA polymerase mutant is obtained.

The above method of introducing the site mutation by using each primer pair shown in Table 1 is as follows.

The recombinant vector WT is tested as a template, in a site-directed mutation PCR reaction system (25 μl) containing the following components: 2.5 μl 10x Pfu Reaction Buffer with $Mg^{2+}$, 2 μl dNTP Mix (2.5 mM each), 25 ng pET28a-Phi29 plasmid, 0.5 μl Pfu DNA Polymerase, mutation primers of mutation sites (Table 1) are respectively added, and PCR amplification is performed.

PCR conditions are as follows: 95°C 3 min, 19 cycle for [95°C 30s, 53°C 30s, 68°C 8 min], 68°C 10 min, 4°C forever. After the reaction product is digested with DpnI, it is transformed into DH5a competent cells, spread out on a kanamycin-resistant LB plate, and then it is incubated overnight in an incubator at 37°C, a monoclonal plasmid is picked out for sequencing, to verify whether a specific amino acid site is successfully mutated.

Table 1: Single-site mutation primers

| Phi29 DNA polymerase mutant | Forward primer | Reverse primer |
|---|---|---|
| M97H | CACCATTATTAGCCGCCATGGCCAGT GGTA TATGATTG | CAATCATATACCACTGGCCATGGCGGCT AATA ATGGTG |
| M97A | CACCATTATTAGCCGCGCGGCCAGT GGTA TATGATTG | CAATCATATACCACTGGCCCGCGCGGC TAATA ATGGTG |
| M97K | CACCATTATTAGCCGCAAAGGCCAGT GGTA TATGATTG | CAATCATATACCACTGGCCTTTGCGGCT AATA ATGGTG |
| L123K | CCGTGATCTATGATAGCAAAAAGAAAC TGC CGTTTCCG | CGGAAACGGCAGTTTCTTTTTGCTATCA TAGA TCACGG |
| L123F | CCGTGATCTATGATAGCTTTAAGAAAC TGC CGTTTCCG | CGGAAACGGCAGTTTCTTAAAGCTATCA TAGA TCACGG |
| L123I | CCGTGATCTATGATAGCATTAAGAAAC TGC CGTTTCCG | CGGAAACGGCAGTTTCTTAATGCTATCA TAGA TCACGG |
| L123H | CCGTGATCTATGATAGCCATAAGAAAC TGC CGTTTCCG | CGGAAACGGCAGTTTCTTATGGCTATCA TAGA TCACGG |
| E515P | GGATGGCAAACTGGTTCCGGGCAGC CCGGA TG | CATCCGGGCTGCCCGGAACCAGTTTGC CATCC |
| Phi29-192 (Y224K) | GATAAAGAAGTGCGCAAAGCGTATCG CGGT GGC | GCCACCGCGATACGCTTTGCGCACTTC TTTATC |
| Phi29-103 (G217E) | GACCCTGAGCCTGGAACTGGATAAAG AAGT GC | GCACTTCTTTATCCAGTTCCAGGCTCAG GGTC |
| phi29-85 (I474K) | GATGTGATCAAAGATTTTGTGGACCC GAAA AAAC | GTTTTTTCGGGTCCACAAAATCTTTGAT CACA TC |

Compared with the recombinant vector WT, a difference from a recombinant vector for expressing a Phi29 DNA polymerase mutant M97H is only that: the 289-291-th nucleotides of the DNA molecule shown in SEQ ID NO: 1 of the sequence listing are mutated from "ATG" into "CAT". The mutated DNA molecule encodes the mutant M97H. Compared with the wild-type Phi29 DNA polymerase, a difference from the mutant M97H is only that the amino acid residue in the 97-th site of the wild-type Phi29 DNA polymerase amino acid sequence is mutated from M into H.

Compared with the recombinant vector WT, a difference from a recombinant vector for expressing a Phi29 DNA polymerase mutant M97A is only that: the 289-291-th nucleotides of the DNA molecule shown in SEQ ID NO: 1 of the sequence listing are mutated from "ATG" into "GCG". The mutated DNA molecule encodes the mutant M97A. Compared with the wild-type Phi29 DNA polymerase, a difference from the mutant M97A is only that the amino acid residue in the 97-th site of the wild-type Phi29 DNA polymerase amino acid sequence is mutated from M into A.

Compared with the recombinant vector WT, a difference from a recombinant vector for expressing a Phi29 DNA polymerase mutant M97K is only that: the 289-291-th nucleotides of the DNA molecule shown in SEQ ID NO: 1 of the sequence listing are mutated from "ATG" into "AAA". The mutated DNA molecule encodes the mutant M97K. Compared with the wild-type Phi29 DNA polymerase, a difference from the mutant M97K is only that the amino acid residue in the 97-th site of the wild-type Phi29 DNA polymerase amino acid sequence is mutated from M into K.

Compared with the recombinant vector WT, a difference from a recombinant vector for expressing a Phi29 DNA polymerase mutant L123K is only that: the 367-369-th nucleotides of the DNA molecule shown in SEQ ID NO: 1 of the sequence listing are mutated from "CTG" into "AAA". The mutated DNA molecule encodes the mutant L123K. Compared with the wild-type Phi29 DNA polymerase, a difference from the mutant L123K is only that the amino acid residue in the 123-th site of the wild-type Phi29 DNA polymerase amino acid sequence is mutated from L into K.

Compared with the recombinant vector WT, a difference from a recombinant vector for expressing a Phi29 DNA polymerase mutant L123F is only that: the 367-369-th nucleotides of the DNA molecule shown in SEQ ID NO: 1 of the sequence listing are mutated from "CTG" into "TTT". The mutated DNA molecule encodes the mutant L123F. Compared with the wild-type Phi29 DNA polymerase, a difference from the mutant L123F is only that the amino acid residue in the 123-th site of the wild-type Phi29 DNA polymerase amino acid sequence is mutated from L into F.

Compared with the recombinant vector WT, a difference from a recombinant vector for expressing a Phi29 DNA polymerase mutant L123I is only that: the 367-369-th nucleotides of the DNA molecule shown in SEQ ID NO: 1 of the sequence listing are mutated from "CTG" into "ATT". The mutated DNA molecule encodes the mutant L123I. Compared with the wild-type Phi29 DNA polymerase, a difference from the mutant L123I is only that the amino acid residue in the 123-th site of the wild-type Phi29 DNA polymerase amino acid sequence is mutated from L into I.

Compared with the recombinant vector WT, a difference from a recombinant vector for expressing a Phi29 DNA polymerase mutant L123H is only that: the 367-369-th nucleotides of the DNA molecule shown in SEQ ID NO: 1 of the sequence listing are mutated from "CTG" into "CAT". The mutated DNA molecule encodes the mutant L123H. Compared with the wild-type Phi29 DNA polymerase, a difference from the mutant L123H is only that the amino acid residue in the 123-th site of the wild-type Phi29 DNA polymerase amino acid sequence is mutated from L into H.

Compared with the recombinant vector WT, a difference from a recombinant vector for expressing a Phi29 DNA polymerase mutant E515P is only that: the 1543-1545-th nucleotides of the DNA molecule shown in SEQ ID NO: 1 of the sequence listing are mutated from "GAA" into "CCG". The mutated DNA molecule encodes the mutant E515P. Compared with the wild-type Phi29 DNA polymerase, a difference from the mutant E515P is only that the amino acid residue in the 515-th site of the wild-type Phi29 DNA polymerase amino acid sequence is mutated from E into P.

Compared with the recombinant vector WT, a difference from a recombinant vector for expressing a Phi29 DNA polymerase mutant Y224K is only that: the 670-672-th nucleotides of the DNA molecule shown in SEQ ID NO: 1 of the sequence listing are mutated from "TAT" into "AAA". The mutated DNA molecule encodes the mutant Y224K. Compared with the wild-type Phi29 DNA polymerase, a difference from the mutant Y224K is only that the amino acid residue in the 224-th site of the wild-type Phi29 DNA polymerase amino acid sequence is mutated from Y into K.

Compared with the recombinant vector WT, a difference from a recombinant vector for expressing a Phi29 DNA polymerase mutant G217E is only that: the 649-651 -th nucleotides of the DNA molecule shown in SEQ ID NO: 1 of the sequence listing are mutated from "GGC" into "GAA". The mutated DNA molecule encodes the mutant G217E. Compared with the wild-type Phi29 DNA polymerase, a difference from the mutant G217E is only that the amino acid residue in the 217-th site of the wild-type Phi29 DNA polymerase amino acid sequence is mutated from G into E.

Compared with the recombinant vector WT, a difference from a recombinant vector for expressing a Phi29 DNA polymerase mutant 1474K is only that: the 1420-1422-th nucleotides of the DNA molecule shown in SEQ ID NO: 1 of the sequence listing are mutated from "ATT" into "TTT". The mutated DNA molecule encodes the mutant I474K. Compared with the wild-type Phi29 DNA polymerase, a difference from the mutant I474K is only that the amino acid residue in the 474-th site of the wild-type Phi29 DNA polymerase amino acid sequence is mutated from I into K.

2. Construction of recombinant vector for expressing Phi29 DNA polymerase multi-site mutant

The recombinant vector WT is served as an original vector, each primer pair shown in Table 1 is used to introduce a site mutation successively, to obtain each recombinant vector for expressing a Phi29 DNA polymerase mutant. A specific mutation form is shown in Table 2:

Table 2

| Phi29 DNA polymerase mutant number | Phi29 DNA polymerase mutant name |
|---|---|
| phi29-337 | M97K-L123H-E515P-Y224K-G217E |
| phi29-414 | M97T-L123K-E515P |
| phi29-456 | M97K-L123H-E515P |
| Phi29-464 | Y224K-I474K-E515P |
| phi29-458 | M97K-L123H-E515P-G217E |
| phi29-459 | M97K-L123H-E515P-Y224K |
| Phi29-463 | Y224K-I474K |
| Phi29-335 | Y224K-G217E |

Compared with the recombinant vector WT, a difference from a recombinant vector for expressing a Phi29 DNA polymerase mutant M97K-L123H-E515P-Y224K-G217E is only that: the 289-291-th nucleotides of the DNA molecule shown in SEQ ID NO: 1 of the sequence listing are mutated from "ATG" into "AAA", the 367-369-th nucleotides of the DNA molecule shown in SEQ ID NO: 1 of the sequence listing are mutated from "CTG" into "CAT", the 1543-1545-th nucleotides of the DNA molecule shown in SEQ ID NO: 1 of the sequence listing are mutated from "GAA" into "CCG", the 670-672-th nucleotides of the DNA molecule shown in SEQ ID NO: 1 of the sequence listing are mutated from "TAT" into "AAA", the 649-651-th nucleotides of the DNA molecule shown in SEQ ID NO: 1 of the sequence listing are mutated from "GGC" into "GAA". The mutated DNA molecule encodes the mutant M97K-L123H-E515P-Y224K-G217E. Compared with the wild-type Phi29 DNA polymerase, a difference from the mutant M97K-L123H-E515P-Y224K-G217E is only that the amino acid residue in the 97-th site of the wild-type Phi29 DNA polymerase amino acid sequence is mutated from M into K, and the 123-th site is mutated from L into H, the 515-th site is mutated from E into P, the 224-th site is mutated from Y into K, and the 217-th site is mutated from G into E.

Compared with the recombinant vector WT, a difference from a recombinant vector for expressing a Phi29 DNA polymerase mutant M97T-L123K-E515P is only that the 289-291-th nucleotides of the DNA molecule shown in SEQ ID NO: 1 of the sequence listing are mutated from "ATG" into "ACC", the 367-369-th nucleotides of the DNA molecule shown in SEQ ID NO: 1 of the sequence listing are mutated from "CTG" into "AAA", the 1543-1545-th nucleotides of the DNA molecule shown in SEQ ID NO: 1 of the sequence listing are mutated from "GAA" into "CCG". The mutated DNA molecule encodes the mutant M97T-L123K-E515P. Compared with the wild-type Phi29 DNA polymerase, a difference from the mutant M97T-L123K-E515P is only that the amino acid residue in the 97-th site of the wild-type Phi29 DNA polymerase amino acid sequence is mutated from M into T, and the 123-th site is mutated from L into K, and the 515-th site is mutated from E into P.

Compared with the recombinant vector WT, a difference from a recombinant vector for expressing a Phi29 DNA polymerase mutant M97K-L123H-E515P is only that: the 289-291-th nucleotides of the DNA molecule shown in SEQ ID NO: 1 of the sequence listing are mutated from "ATG" into "AAA", the 367-369-th nucleotides of the DNA molecule shown in SEQ ID NO: 1 of the sequence listing are mutated from "CTG" into "CAT", the 1543-1545-th nucleotides of the DNA molecule shown in SEQ ID NO: 1 of the sequence listing are mutated from "GAA" into "CCG". The mutated DNA molecule encodes the mutant M97T-L123H-E515P. Compared with the wild-type Phi29 DNA polymerase, a difference from the mutant M97K-L123H-E515P is only that the amino acid residue in the 97-th site of the wild-type Phi29 DNA polymerase amino acid sequence is mutated from M into T, and the 123-th site is mutated from L into H, and the 515-th site is mutated from E to P.

Compared with the recombinant vector WT, a difference from a recombinant vector for expressing a Phi29 DNA polymerase mutant Y224K-I474K-E515P is only that: the 670-672-th nucleotides of the DNA molecule shown in SEQ ID NO: 1 of the sequence listing are mutated from "TAT" into "AAA", the 1420-1422-th nucleotides of the DNA molecule shown in SEQ ID NO: 1 of the sequence listing are mutated from "ATT" into "TTT", the 1543-1545-th nucleotides of the DNA molecule shown in SEQ ID NO: 1 of the sequence listing are mutated from "GAA" into "CCG". The mutated DNA molecule encodes the mutant Y224K-I474K-E515P. Compared with the wild-type Phi29 DNA polymerase, a difference from the mutant Y224K-I474K-E515P is only that the amino acid residue in the 224-th site of the wild-type Phi29 DNA polymerase amino acid sequence is mutated from Y into K, and the 474-th site is mutated from I into K, and the 515-th site is mutated from E into P.

Compared with the recombinant vector WT, a difference from a recombinant vector for expressing a Phi29 DNA polymerase mutant M97K-L123H-E515P-G217E is only that: the 289-291 -th nucleotides of the DNA molecule shown in SEQ ID NO: 1 of the sequence listing are mutated from "ATG" into "AAA", the 367-369-th nucleotides of the DNA molecule shown in SEQ ID NO: 1 of the sequence listing are mutated from "CTG" into "CAT", the 1543-1545-th nucleotides of the DNA molecule shown in SEQ ID NO: 1 of the sequence listing are mutated from "GAA" into "CCG", and the 649-651-th nucleotides of the DNA molecule shown in SEQ ID NO: 1 of the sequence listing are mutated from "GGC" to "GAA". The mutated DNA molecule encodes the mutant M97K-L123H-E515P-G217E. Compared with the wild-type Phi29 DNA polymerase, a difference from the mutant M97K-L123H-E515P-G217E is only that the amino acid residue in the 97-th site of the wild-type Phi29 DNA polymerase amino acid sequence is mutated from M into K, the amino acid residue in the 123-th site is mutated from L into H, and the 515-th site is mutated from E into P, and the 217-th site is mutated from G into E.

Compared with the recombinant vector WT, a difference from a recombinant vector for expressing a Phi29 DNA polymerase mutant M97K-L123H-E515P-Y224K is only that: the 289-291-th nucleotides of the DNA molecule shown in SEQ ID NO: 1 of the sequence listing are mutated from "ATG" into "AAA", the 367-369-th nucleotides of the DNA molecule shown in SEQ ID NO: 1 of the sequence listing are mutated from "CTG" into "CAT", the 1543-1545-th nucleotides of the DNA molecule shown in SEQ ID NO: 1 of the sequence listing are mutated from "GAA" into "CCG", and the 670-672-th nucleotides of the DNA molecule shown in SEQ ID NO: 1 of the sequence listing are mutated from "TAT" into "AAA". The mutated DNA molecule encodes the mutant M97K-L123H-E515P-G217E. Compared with the wild-type Phi29 DNA polymerase, a difference from the mutant M97K-L123H-E515P-G217E is only that the amino acid residue in the 97-th site of the wild-type Phi29 DNA polymerase amino acid sequence is mutated from M into K, the amino acid residue in the 123-th site is mutated from L into H, and the 515-th site is mutated from E into P, and the 224-th site is mutated from Y into K.

Compared with the recombinant vector WT, a difference from a recombinant vector for expressing a Phi29 DNA polymerase mutant Y224K-I474K is only that: the 670-672-th nucleotides of the DNA molecule shown in SEQ ID NO: 1 of the sequence listing are mutated from "TAT" into "AAA", the 1420-1422-th nucleotides of the DNA molecule shown in SEQ ID NO: 1 of the sequence listing are mutated from "ATT" into "TTT". The mutated DNA molecule encodes the mutant Y224K-I474K. Compared with the wild-type Phi29 DNA polymerase, a difference from the mutant Y224K-I474K is only that the amino acid residue in the 224-th site of the wild-type Phi29 DNA polymerase amino acid sequence is mutated from Y into K, and the amino acid residue in the 474-th site is mutated from I into K.

II. Construction of recombinant bacteria for expressing Phi29 DNA polymerase mutant

The recombinant vector WT and each recombinant vector for expressing the Phi29 DNA polymerase mutant constructed in step I are respectively introduced into *Escherichia coli* BL21 (DE3), to obtain recombinant bacteria for expressing the wild-type Phi29 DNA polymerase and each recombinant bacterium for expressing the Phi29 DNA polymerase mutant.

III. Induced expression of recombinant bacteria

The recombinant bacteria for expressing the wild-type Phi29 DNA polymerase and each recombinant bacterium for expressing the Phi29 DNA polymerase mutant obtained in step II are taken, and sequentially induced and purified, to obtain the wild-type Phi29 DNA polymerase fused with an $HiS_6$ tag at an N-terminal and each Phi29 DNA polymerase mutant fused with an $HiS_6$ tag at an N-terminal.

The wild-type Phi29 DNA polymerase fused with the $HiS_6$ tag at the N-terminal and each Phi29 DNA polymerase mutant fused with the $HiS_6$ tag at the N-terminal are successively named as a wild-type Phi29 DNA polymerase with the $HiS_6$ tag, a Phi29 DNA polymerase mutant M97H with the $HiS_6$ tag, a Phi29 DNA polymerase mutant M97A with the $HiS_6$ tag, a Phi29 DNA polymerase mutant M97K with the $HiS_6$ tag, a Phi29 DNA polymerase mutant L123K with the $HiS_6$ tag, a Phi29 DNA polymerase mutant L123F with the $HiS_6$ tag, a Phi29 DNA polymerase mutant L123I with the $HiS_6$ tag, a Phi29 DNA polymerase mutant L123H with the $HiS_6$ tag, a Phi29 DNA polymerase mutant E515P with the $HiS_6$ tag, a Phi29 DNA polymerase mutant E515G with the $HiS_6$ tag, a Phi29 DNA polymerase mutant Y224K with the $HiS_6$ tag, a Phi29 DNA polymerase mutant G217E with the $HiS_6$ tag, a Phi29 DNA polymerase mutant I474K with the $HiS_6$ tag, a Phi29 DNA polymerase mutant M97K-L123H-E515P-Y224K-G217E with the $His_6$ tag, a Phi29 DNA polymerase mutant M97T-L123K-E515P with the $His_6$ tag, a Phi29 DNA polymerase mutant Y224K-I474K-E515P with the $HiS_6$ tag, a Phi29 DNA polymerase mutant M97K-L123H-E515P-G217E with the $HiS_6$ tag, a Phi29 DNA polymerase mutant M97K-L123H-E515P-Y224K with the $HiS_6$ tag, a Phi29 DNA polymerase mutant Y224K-I474K with the $HiS_6$ tag,

and a Phi29 DNA polymerase mutant Y224K-G217E with the $His_6$ tag.

1. Specific steps of induction are as follows:

(1) Live bacteria

The recombinant bacteria are seeded into 3 ml of a liquid LB medium containing Kana resistance, and cultured overnight.

(2) Transfer

After the step (1) is completed, the above bacterial solution is transferred to 2 ml of the liquid LB medium containing the Kana resistance according to a 1/100 volume, and shake-cultured at 37°C and 220 rpm until $OD_{600nm}$ = 0.6 (in a practical application, $OD_{600nm}$ = 0.4-0.8 is acceptable).

(3) Induction

After the step (2) is completed, IPTG with a final concentration of 0.5 mM is added to a system, and shake-cultured at 16°C and 220 rpm for 12 h.

(4) Bacteria collection

After the step (3) is completed, it is centrifuged at 4°C and 8000 rpm for 5 min, and bacteria are collected.

2. An AKTA Pure purification system of GE Company is used for purification, and specific steps are as follows:

(1) The bacteria obtained in the step 1 are taken, shaken and uniformly mixed with resuspension buffer (20 mM Tris-HCl, 500 mM NaCl, 20 mM Imidazole, 5% Glycerol, and pH 7.9 @25°C), it is ultrasonically crushed on ice, and then centrifuged at 4°C and 12000 rpm for 30 min, and supernatant is collected.

(2) The supernatant obtained in the step (1) is taken, and a nickel column affinity chromatography (HisTrap FF 5 ml pre-packed column) is used for purification. Specific steps are as follows: firstly it is equilibrated with 10 column volumes of Buffer A; then a sample is loaded; then it is rinsed with 20 column volumes of the Buffer A; then it is eluted with 15 column volumes of eluent and post-column solution with a target protein is collected (the eluent is formed by Buffer A and Buffer B, and in an elution process, a volume fraction of the Buffer B is linearly increased from 0% to 100%, and correspondingly a volume fraction of the Buffer A is linearly decreased 100% to 0%).

Buffer A: 20 mM Tris-HCl, 500 mM NaCl, 20 mM Imidazole, 5% (v/v) Glycerol; pH 7.9@ 25°C.

Buffer B: 20 mM Tris-HCl, 500 mM NaCl, 500 mM Imidazole, 5% (v/v) Glycerol; pH7.9@ 25°C.

(3) The post-column solution obtained in the step (2) is taken, and a strong anion column chromatography (HiTrap Q HP 5 ml pre-packed column) is used for purification. Specific steps are as follows: firstly it is equilibrated with 10 column volumes of mixed buffer formed by Buffer A with a volume fraction of 59% and Buffer B with a volume fraction of 41%; then a sample is loaded; and after a protein peak appears, flow-through solution is collected (collection is performed after a UV detection value raises to 20 mAu, and the collection is stopped after the UV detection value drops to 50 mAu).

Buffer A: 20 mM Tris-HCl, 150 mM NaCl, 5% (v/v) Glycerol, pH7.5@ 25°C.

Buffer B: 20 mM Tris-HCl, 1 M NaCl, 5% (v/v) Glycerol, pH7.5@ 25°C.

(4) The flow-through solution obtained in the step (3) is taken, and a cation exchange chromatography (HiTrap SP HP pre-packed column) is used for purification, to obtain protein sample solution of which a purity is greater than 95%. Specific steps are as follows: firstly it is equilibrated with 10 column volumes of Buffer A; then a sample is loaded; then it is rinsed with 15 column volumes of the Buffer A; then it is eluted with 10 column volumes of eluent (the eluent is formed by Buffer A and Buffer B, and in an elution process, a volume fraction of the Buffer B is linearly increased from 0% to 50%, and correspondingly a volume fraction of the Buffer A is linearly decreased 100% to 50%) and post-column solution with a target protein is collected (collection is performed after a UV detection value raises to 50 mAu, and the collection is stopped after the UV detection value drops to 100 mAu).

Buffer A: 20 mM Tris-HCl, 150 mM NaCl, 5% (v/v) Glycerol, pH7.5@ 25°C.

Buffer B: 20 mM Tris-HCl, 1 M NaCl, 5% (v/v) Glycerol, pH7.5@ 25°C.

(5) The target protein collected in the step (4) is taken and transferred to a dialysis bag, after dialysis in dialysis buffer overnight, protein solution in the dialysis bag is collected, and other reagents are added, to obtain target protein solution with a protein concentration of 1 mg/ml. The concentrations of other components in the target protein solution are as follows: 10 mM Tris-HCl (pH7.4@ 25°C), 100 mM KCl, 1 mM DTT, 0.1 mM EDTA, 0.5%(v/v)NP-40, 0.5% (v/v) Tween20, 50% (v/v) Glycerol.

Dialysis buffer: 23.75 mM Tris-HCl (pH7.4@ 25°C), 237.5 mM KCl, 2.375 mM DTT, 0.2375 mM EDTA, 5% (v/v) Glycerol.

Embodiment 2: Polymerization activity test of wild-type Phi29 DNA polymerase and mutant

The wild-type Phi29 DNA polymerase solution with the $HiS_6$ tag and the Phi29 DNA polymerase mutant solution with the $HiS_6$ tag prepared in Embodiment 1 are taken as enzyme solution to be tested.

1. Each enzyme solution to be tested is diluted to 5000 times in a gradient by using storage buffer, adequately and uniformly mixed in a vortex shaker, and it is standing on ice for 5 min, to obtain each solution to be tested.
A pre-reaction system is uniformly mixed in a PCR tube, and placed in a PCR instrument, the following procedures are performed: 95°C 1 min, 65°C 1 min, 40°C 1 min, and a hot lid temperature is set to 102°C.
Pre-reaction system (80.8 μl): 50 mM Tris-HCl (pH7.5), 4 mM DTT, 10 mM $(NH_4)_2SO_4$, 10 mM $MgCl_2$, 50 nM dNTP Mixture, 2 pM 141RCA Primer and 18 ng single-stranded circular DNA template 141 Ad ssDNA.

2. After the step 1 is completed, the PCR tube is taken out and placed on ice while the temperature reaches 4°C. 1 μl of each solution to be tested is respectively added in a test group, and 1 μl of the storage buffer is added in a negative control group. Then, the vortex shaker is used to shake and mix uniformly, it is placed in the PCR instrument after being transitorily centrifuged for 5 s in a centrifuge, and the following procedures are performed: 30°C 60 min, and a hot lid temperature is set to 65°C.

3. After the step 2 is completed, 5 μL of 0.5 M EDTA solution is added to stop a reaction, and it is shaken and mixed uniformly.

4. A Qubit ssDNA Assay Kit (Q10212, INVITROGEN) is used and operated according to instructions. A Qubit fluorometer3.0 is used to detect a concentration of DNB (DNA Nano ball) in a reaction product.

Enzyme activity of enzyme solution to be tested=ΔDNBx5000÷37.38.

Note: ΔDNB is a difference value between concentration average values of the reaction products in the systems after the reactions of the test group and the negative control group are terminated, 5000 is a dilution factor, and 37.38 is a slope of a functional relationship between the enzyme activity and the ΔDNB.

Enzyme activity results of some enzyme solution to be tested are shown in Table 3.

Table 3

| Polymerase | Control group average value | Experimental group average value | ΔDNB (ng/μl) | Enzyme activity of prepared enzyme solution (U/μl) |
|---|---|---|---|---|
| WT | 0.46 | 0.96 | 0. 51 | 67.6 |
| E515P | 0.37 | 0. 73 | 0. 37 | 49.3 |
| L123K | 0.4 | 0. 77 | 0. 37 | 50.1 |

(continued)

| Polymerase | Control group average value | Experimental group average value | ΔDNB (ng/μl) | Enzyme activity of prepared enzyme solution (U/μl) |
|---|---|---|---|---|
| M97A | 0.4 | 0.75 | 0.36 | 48 |
| G217E | 0.42 | 0.97 | 0.55 | 73.8 |
| Y224K | 0. 38 | 0.8 | 0.42 | 56.1 |
| 1474K | 0.48 | 1.06 | 0.57 | 15.4 |

Embodiment 3: Thermal stability test of wild-type Phi29 DNA polymerase and mutant

37°C 10 min experiment:

The target protein solution (the wild-type Phi29 DNA polymerase solution with the $His_6$ tag and the Phi29 DNA polymerase mutant solution with the $His_6$ tag) prepared in Embodiment 1 is taken, and divided into two parts, and they are respectively treated as follows:

First part: it is placed in a metal bath preheated to 37°C for 10 min, and centrifuged at 4°C and 13000 rpm for 1 min, and supernatant is collected; and then, the supernatant is taken, and diluted to 1000 times of a volume with the storage buffer, and the vortex shaker is used to adequately mix uniformly, and then it is standing on ice for 5 min, to obtain solution 1 to be tested.

Second part: it is diluted to 5000 times of a volume with the storage buffer, and adequately mixed uniformly with the vortex shaker, and then it is standing on ice for 5 min, to obtain solution 2 to be tested.

It is performed according to the steps 1 to 4 of Embodiment 2.

Unheated enzyme activity (U1)=ΔDNB×5000÷37.38; ΔDNB is a difference value between concentration average values of reaction products in the systems after the reactions of the test group (second part) and the negative control group are terminated;

Heated enzyme activity (U2)=ΔDNB×1000÷37.38; ΔDNB is a difference value between concentration average values of reaction products in the systems after the reactions of the test group (first part) and the negative control group are terminated;

5000 and 1000 are dilution factors respectively, and 37.38 is a slope of a functional relationship between the enzyme activity and the ΔDNB;

an enzyme activity loss ratio is calculated according to the activities of the unheated and heated enzyme solution,

$$\text{Enzyme activity loss ratio (\%)}=(U1-U2)\div U1\times 100\%;$$

U1 is the activity of the unheated enzyme solution, and U2 is the activity of the heated enzyme solution.

-20°C experiment: a difference from the 37°C 10 min experiment is only that the 10 min in the 37°C metal bath is replaced with the 10 min in a refrigerator at -20°C.

The specific enzyme activity and the enzyme activity loss ratio of each mutant are shown in Table 4. Compared with the wild type (WT), the enzyme activity loss ratios of the mutants in examples are all improved to a certain extent; and a numeral value of the enzyme activity loss ratio is larger, the enzyme is more unstable, and the thermal stability is worse.

Table 4

| Mutation site | Heat treatment condition | Average value (ng/ μl) | ΔDNB (ng/ μl) | Diluted enzyme solution activity (U) | Originalenzyme solution activity (U/ μl) | Specific enzyme activity (U/ ug) | Enzyme activity loss ratio | Note: Enzyme concentration (mg/ ml) | Note: Original enzyme solution dilution factor |
|---|---|---|---|---|---|---|---|---|---|
| Phi29-WT | -20°C | 1.12 | 0.74 | 0.02 | 59.2 | 74 | 98.60 % | 0.8 | 3000 |
| (Wild type) | 37 °C 10min | 0.68 | 0.3 | 0.008 | 0.8 | | | | 100 |
| | NC | 0.38 | | | | | | | |
| Phi29-Enzymatics (Commercial enzyme of Enzymatics Company) | -20 °C | 1.04 | 0.57 | 0.015 | 76.7 | 63.9 | 85.40 % | 1.2 | 5000 |
| | 37 °C 10min | 0.55 | 0.08 | 0.002 | 11.2 | | | | 5000 |
| | NC | 0.46 | | | | | | | |
| Phi29-337 (M97K-L123H-E515P-Y224K-G217E) | -20 °C | 1.87 | 1.41 | 0.038 | 18.8 | 104.6 | 7.80% | 0.18 | 500 |
| | 37 °C 15min | 1.76 | 1.3 | 0.035 | 17.4 | | | | 500 |
| | NC | 0.46 | | | | | | | |
| Phi29-414 | -20 °C | 1.13 | 0.734 | 0.020 | 19.6 | 67.7 | 12.26 % | 0.29 | 1000 |
| (M97T-L123K-E515P) | 37 °C 10min | 1.04 | 0.644 | 0.017 | 17.2 | | | | 1000 |
| | NC | 0.396 | | | | | | | |
| Phi29-456 | -20 °C | 1.14 | 0.697 | 0.018 | 36.9 | 60.2 | 35.19 % | 0.613 | 2000 |
| (M97K-L123H-E515P) | 37 °C 10min | 2.25 | 1.807 | 0.048 | 23.9 | | | | 500 |
| | NC | 0.443 | | | | | | | |
| Phi29-464 | -20 °C | 2.05 | 1.633 | 0.027 | 53.72 | 57.15 | 63.67 % | 0.94 | 2000 |
| (Y224K-I474K-E515P) | 37 °C 10min | 2.79 | 2.373 | 0.039 | 19.51 | | | | 500 |
| | NC | 0.417 | | | | | | | |

(continued)

| Mutation site | Heat treatment condition | Average value (ng/ μl) | ΔDNB (ng/ μl) | Diluted enzyme solution activity (U) | Originalenzyme solution activity (U/ μl) | Specific enzyme activity (U/ ug) | Enzyme activity loss ratio | Note: Enzyme concentration (mg/ ml) | Note: Original enzyme solution dilution factor |
|---|---|---|---|---|---|---|---|---|---|
| Phi29-36 | -20 °C | 1.46 | 1.04 | 0.017 | 51.5 | 51.5 | 70.20 % | 1 | 3000 |
| | 37 °C | | | | | | | | |
| (E515P) | 10min | 1.35 | 0.93 | 0.015 | 15.3 | | | | 1000 |
| | NC | 0.42 | | | | | | | |
| Phi29-458 | -20 °C | 0.936 | 0.583 | 0.016 | 31.2 | 31.2 | 70.97 % | 1 | 2000 |
| (M97K-L123H-E515P-G217E) | 37 °C | | | | | | | | |
| | 10min | 1.03 | 0.677 | 0.018 | 9.1 | | | | 500 |
| | NC | 0.353 | | | | | | | |
| Phi29-459 | -20 °C | 0.825 | 0.472 | 0.013 | 25.3 | 25.3 | 72.35 % | 1 | 2000 |
| | 37 °C | | | | | | | | |
| (M97K-L123H -E515P-Y224K) | 10min | 0.875 | 0.522 | 0.014 | 7.0 | | | | 500 |
| | NC | 0.353 | | | | | | | |
| Phi29-463 | -20 °C | 1.81 | 1.347 | 0.04 | 36.04 | 72.07 | 80.91 % | 0.5 | 1000 |
| (Y224K-I474K) | 37 °C | | | | | | | | |
| | 10min | 1.32 | 0.857 | 0.02 | 6.88 | | | | 300 |
| | NC | 0.463 | | | | | | | |
| Phi29-103 | -20 °C | 0.97 | 0.55 | 0.015 | 73.8 | 70.3 | 85.20 % | 1.05 | 5000 |
| | 37 °C | | | | | | | | |
| (G217E) | 10min | 0.5 | 0.08 | 0.002 | 10.9 | | | | 5000 |
| | NC | 0.42 | | | | | | | |
| Phi29-335 | -20 °C | 1.94 | 1.51 | 0.041 | 81 | 85.3 | 86.70 % | 0.95 | 2000 |
| (Y224K-G217E) | 37 °C | | | | | | | | |
| | 10min | 1.23 | 0.8 | 0.022 | 10.8 | | | | 500 |
| | NC | 0.43 | | | | | | | |
| Phi29-192 | -20 °C | 1.43 | 1.05 | 0.028 | 56.1 | 56.1 | 87.30 % | 1 | 2000 |

(continued)

| Mutation site | Heat treatment condition | Average value (ng/ μl) | ΔDNB (ng/ μl) | Diluted enzyme solution activity (U) | Originalenzyme solution activity (U/ μl) | Specific enzyme activity (U/ ug) | Enzyme activity loss ratio | Note | |
|---|---|---|---|---|---|---|---|---|---|
| | | | | | | | | Enzyme concentration (mg/ ml) | Original enzyme solution dilution factor |
| (Y224K) | 37 °C 10min | 1.71 | 1.33 | 0.036 | 7.1 | | | | 200 |
| | NC | 0.38 | | | | | | | |
| Phi29-85 | -20 °C | 0.682 | 0.2205 | 0.027 | 29.5 | 36.9 | 88.66 % | 0.8 | 5000 |
| (I474K) | 37 °C 10min | 0.5865 | 0.125 | 0.039 | 3.3 | | | | 1000 |
| | NC | 0.4615 | | | | | | | |

In the above table, NC is a background value, and a blank is because there is no data calculation processing.

Embodiment 4: Genomic amplification coverage detection

In order to detect a coverage to which a sample is extended before on-machine sequencing, a multiple PCR mode is used to amplify a multiple housekeeping gene in an MDA product.

A specific operating process is as follows: a NA12878 reference genome sample (Coriell Institute) is added to 200 μl of a PCR tube, a total volume is 4 μl, and if it is not enough, PBS is added to supplement to 4 μl. 3 μl of Buffer D2 (QIAGEN REPLI-g Single Cell Kit) is added to the PCR tube, a pipette tip should be attached to a tube wall, and is not inserted under a liquid surface, and centrifuging is performed without shaking. After being centrifuged, it is put on a PCR instrument at 65°C for 10 min. After it is finished, it is placed on ice, 3 μl of neutralization buffer (QIAGEN REPLI-g Single Cell Kit) is added to the PCR tube, the pipette tip should be attached to the tube wall, and is not inserted under the liquid surface, the centrifuging is performed without shaking. 39 μl of MDA reaction buffer (10x phi29 Reaction Buffer 5 μl, 25 mM dNTP 2μl, 1 mM N8Primer 2μl, 100x BSA 0.5 $_1$-11, 10% F68 0.05μl, and $H_2O$ 29.75 μl) is added to the PCR tube, 1 μl of phi29-337, phi29-456, phi29-458, phi29-36, phi29-335, phi29-414, and phi29-464 prepared in Embodiment 1 and 1 μl of the Phi29 DNA polymerase carried by the QIAGEN REPLI-g Single Cell Kit are respectively added. The pipette tip should be attached to the tube wall, and is not inserted below the liquid surface, the centrifuging is performed without shaking. After being centrifuged, it is put on the PCR instrument at 30°C for 8 h and 65°C for 3 min. After a reaction is finished, the shaking and centrifuging are performed, and a concentration is measured with Qubit BR. After the concentration is measured, a multiple housekeeping gene PCR detection is performed. 50-200 ng of a DNA is taken, a negative control (N) and a positive control (P) should be set, the negative is water, the positive is a NA12878 reference genome without amplification, and an experimental group is set with 3 different samples. Multiple PCR primers and procedures are shown in Table 5 below:

Table 5: Multiple PCR primer and PCR reaction system

| Primer name | Primer sequence |
|---|---|
| 18-F | GGCATCGCTTAGACTCTATGTG |
| 18-R | CTGCCCTTGGCCTAACTAACCT |
| 12-F | GTTCCTCAAGTAGCTCCACGAG |
| 12-R | CGTTAGACTCTGGATCTGGCGT |
| 16-F | CCAGCCAGTTCATGCGTCGGTG |
| 16-R | CCTGACAACTCGCAAGTAGCAC |
| 17-F | GCTCAATGGGGTACTTCAGGT |
| 17-R | GTGGACTTACGTAAAAGGCCC |
| 19-F | TGCTCTGTATGTGAAGATGCCA |
| 19R | TTCCAGGTAAATCCAGCCCAGG |
| 3-F | CAGCCAGTCGGCATCATCCAAC |
| 3-R | GAAAGCCGGATTGCGGTAACAT |
| 8-F | GGATAGCTCTGCCAGGGGAGAG |
| 8-R | TCGTCGCAGTAGAAATACGGCT |
| 22-F | AGATGTCAGGCACGTAGCTCAG |
| 22-R | GGCACGTTGGCGTTTACGATGA |
| Reaction component | Volume |
| 10x PCR Buffer | 3 μl |
| dNTP (2.5mM) | 3.2 μl |
| Primer Mix | 3 μl |
| DNA (50ng/μl) | 1 μl |

(continued)

| Reaction component | Volume |
|---|---|
| 100x BSA (NEB) | 0.2 μl |
| rTaq (5U/μl) | 0.4 μl |
| ddH20 | 19.2 μl |
| Total | 30 μl |

PCR procedure: 95°C 5 min, 35 cycles x[94°C 30 s, 60°C 50 s, 72°C 1 min], 72°C 5 min, 12°C holder.

Results are shown in Fig. 2, compared with the wild-type WT, the amplification coverage of the phi29-337, phi29-456, phi29-458, phi29-36, and phi29-414 is greatly improved, and 8 housekeeping gene products may at least be amplified for 6 or more. For the concentrations of the amplified products, the phi29-337, phi29-456, phi29-458, phi29-36, and phi29-414 are higher than the wild-type WT and a phi29 commercial product from Enzymatics Company.

Embodiment 5: Application effect of wild-type Phi29 DNA polymerase and Phi29 DNA polymerase mutant in single-cell whole genome sequencing

In order to test effects of a wild-type Phi29 DNA polymerase and a mutant in single-cell genome sequencing, library construction is performed respectively on products of a NA12878 trace gDNA (200pg) amplified by the wild-type Phi29 DNA polymerase and the Phi29 DNA polymerase mutant prepared in Embodiment 1, after that, high-depth sequencing is performed; at the same time, the same NA12878 trace gDNA (200pg) amplified by a commercial Qiagen single-cell amplification kit is used for control.

Analysis and mutation detection analysis are performed on sequencing data, a NA12878 standard mutation data set is used to evaluate, and a difference between each mutant and the Qiagen kit is compared. Specific data analysis results are shown in Table 6 and Table 7.

It may be seen that the representation of the mutants Phi29-337, Phi29-456, Phi29-335, and Phi29-414 are comparable to that of the wild-type WT and the QIAGEN kit in mapping rate and coverage, but the representation of the wild-type WT is the worst in terms of duplication rate parameters, the mutants listed in the table are all slightly better than the wild type, herein the representation of the 337 mutant is best, and is comparable to the QIAGEN (REPLI-g Single Cell Kit) kit. At the same time, in aspects of mutation detection evaluation parameters snp_Precision, snp_Sensitivity, and snp_F-measure, the representation of the 337 mutant is best, and slightly better than the QIAGEN kit, the wild-type WT and the 456, 335, 414 mutants are worse than the QIAGEN kit; and in aspects of parameters indel_Precision, indel_Sensitivity and indel_F-measure, the representation of the 337 mutant is also the best, and data is better than the QIAGEN kit.

Table 6: Sequencing data analysis of wild-type Phi29 and mutant in single-cell sequencing

| Mutant number | QIAGEN | Phi29-WT | Phi29-36 | Phi29-335 | Phi29-337 |
|---|---|---|---|---|---|
| ReadLength | 100 | 100 | 100 | 100 | 100 |
| Read_raw | 1.12E+09 | 7.96E+08 | 1.07E+09 | 1.01 E+09 | 1.08E+09 |
| Q30_clean | 88.67% | 87.09% | 86% | 87.67% | 87.07% |
| Depth_clean | 28.48 | 19.96 | 28.95 | 25.56 | 28.47 |
| Mapping rate | 99.93% | 99.85% | 99.95% | 99.91% | 99.93% |
| Duplicate rate | 3.71% | 5.07% | 4.56% | 4.85% | 3.87% |
| Coverage | 98.01 % | 97.20% | 94.52% | 97.16% | 98.33% |
| Genome depth CV | 1.01 | 2.21 | 1.36 | 1.7 | 1 |
| GC depth CV | 0.35 | 0.34 | 0.35 | 0.33 | 0.36 |
| snp_Precision | 0.9222 | 0.8993 | 0.9004 | 0.8747 | 0.9383 |
| snp_Sensitivity | 0.92 | 0.8621 | 0.8199 | 0.8466 | 0.9349 |
| snp_F-measure | 0.9211 | 0.8803 | 0.8582 | 0.8604 | 0.9366 |

(continued)

| Mutant number | QIAGEN | Phi29-WT | Phi29-36 | Phi29-335 | Phi29-337 |
|---|---|---|---|---|---|
| indel_Precision | 0.3466 | 0.1229 | 0.072 | 0.1405 | 0.7437 |
| indel_Sensitivity | 0.7249 | 0.5759 | 0.5076 | 0.5925 | 0.7819 |
| indel_F-measure | 0.469 | 0.2025 | 0.126 | 0.2271 | 0.7623 |

Table 7: Sequencing data analysis of wild-type Phi29 and mutant in single-cell sequencing

| Mutant number | Phi29-414 | Phi29-456 | Phi29-458 |
|---|---|---|---|
| ReadLength | 100 | 100 | 100 |
| Read_raw | 1.14E+09 | 1.28E+09 | 1.25E+09 |
| Q30_clean | 87.90% | 88.54% | 88.02% |
| Depth_clean | 28.98 | 32.99 | 32.74 |
| Mapping rate | 99.91% | 99.91% | 99.96% |
| Duplicate rate | 4.67% | 4.51% | 4.31% |
| Coverage | 97.03% | 97.60% | 95.79% |
| Genome depth CV | 1.09 | 1.01 | 1.06 |
| GC depth CV | 0.35 | 0.35 | 0.36 |
| snp_Precision | 0.9183 | 0.9379 | 0.9335 |
| snp_Sensitivity | 0.8898 | 0.9165 | 0.8707 |
| snp_F-measure | 0.9038 | 0.9271 | 0.901 |
| indel_Precision | 0.1318 | 0.1327 | 0.075 |
| indel_Sensitivity | 0.6275 | 0.6667 | 0.5502 |
| indel_F-measure | 0.2179 | 0.2214 | 0.132 |
| (Note: QIAGEN is a control of a commercial REPLI-g Single Cell Kit) | | | |

Embodiment 6: Detection of RCA library construction on-machine testing effect

Synthesized with the thermal stability results in Table 4, the embodiment selects several mutants with improved thermal stability, and performs an on-machine test in a BGISEQ-500 sequencer, application effects of the different mutants in DNA sequencing are detected with reference to a standard of the BGISEQ-500 sequencer. Reagents used in the whole test are a complete set of a PE50V2.0 kit produced by the BGI, an E. coil Ad153 standard library produced by the BGI and a Qubit ssDNA Assay reagent produced by Invitrogen. The reagents used below are all included in the PE50V2.0 kit except the library and QubitssDNA Assay, and a PE50V2.0 reagent tank written below only refers to reagents used in the on-machine test.

1. DNB preparation

DNB preparation buffer, a standard library, DNB polymerase mixed solution and DNB polymerase mixed solution II are taken out from a refrigerator at -20°C and melted on an ice box, after being completely dissolved, they are placed on a vortex shaker, shaken and continuously mixed uniformly for 5 s, and transiently centrifuged in a handheld centrifuge for 3 s, then it is placed on the ice box for later use. Molecular-grade water and DNB stop buffer are taken out from a refrigerator at 4°C, and placed on the ice box for later use. In a labeled eight-connected tube, 20 μl of the DNB preparation buffer and 6 ng of the ssDNA (E. coli standard substance library) are successively added, and supplemented to 40 μl with Nuclease Free Water. The eight-connected tube is placed in the vortex shaker and continuously mixed uniformly for 5 s, and transiently centrifuged for 3 s in the handheld centrifuge. The above eight-connected tube is placed in the PCR instrument, and reaction conditions are set or checked: 95°C 1 min, 65°C 1 min, 40°C 1 min, and 4°C for maintaining (a hot lid temperature is set to 103°C). After a reaction is completed, the PCR eight-connected tube is taken, and placed on the ice box. After a lid temperature of the eight-connected tube drops to a room temperature, it is placed in the handheld centrifuge and transiently centrifuged for 3 s, and immediately placed on the ice box. Each of the following components is successively added into the eight-connected tube: 40 μl of reaction solution in the eight-connected tube after denaturation, 40 μl of the DNB polymerase mixed solution, and 4 μl of the DNB polymerase mixed solution II. The eight-connected tube is placed in the vortex shaker and continuously mixed uniformly for 5 s, and transiently centrifuged for 3 s in the handheld centrifuge. It is immediately placed in the PCR instrument or a water bath kettle to start a reaction. Reaction conditions are set or checked: 30°C 20min, and 4°C for maintaining (a hot lid does not need to be installed). While the above RCA reaction is completed, the eight-connected tube is taken and placed on the ice box, and 20 μl of the DNB stop buffer is immediately added. A pipette with a 100 μl wide-mouth pipette tip is used to slowly blow and mix uniformly for 5-8 times (one-suction and one-blow are counted as 1 time). After being mixed uniformly, it is stored at 4°C for later use. A Qubit ssDNA Assay Kit and a Qubit Fluorometer instrument are used to measure a concentration of the above DNB preparation product. While the concentration is 8ng/μl-40ng/μl, it is judged to be qualified, and it is reserved for a subsequent experiment.

2. DNB loading

After the DNB is prepared, the DNB needs to be loaded on a chip, namely DNB loading.

A sample loading reagent plate V2.1 is taken and placed at a room temperature for melting, shaken and mixed uniformly. After being transiently centrifuged, it is placed on the ice box for later use. DNB loading buffer II is taken, and shaken uniformly. After being transiently centrifuged, it is placed on the ice box for later use. Firstly, the chip and the sample loading reagent plate V2.1 are placed on BGIDL-50. Secondly, 35 μl of the DNB loading buffer II is taken and added to a PCR tube containing 100 μl of the DNB, and a wide-mouth sucker is used to gently mix uniformly for 15 times. Then, the above mixed solution is placed in a designated DNB placement area of a loading system, a DNB loading program (Sample load 2.0) is selected, and loading is started. Finally, after the loading is completed, it is incubated at the room temperature for 30 min, and then stored at 2-8°C for later use.

3. On-machine test

For the wild-type phi29 polymerase or mutant to be tested, an on-machine sequencing test is performed on the BGISEQ-500 sequencer, one chip and one BGISEQ-500RS high-throughput sequencing reagent tank (PE50V2.0) are used. Before the on-machine test, a sequencing reagent tank II, dNTPs mixed solution (V3.0) and dNTPs mixed solution II (V2.0) are firstly thawed and melted, and placed in a refrigerator or ice box at 4°C for later use; a sequencing enzyme is shaken and mixed uniformly, and placed on the ice box for later use. Firstly, a No. 5 well reagent is prepared, namely 1 ml of a pipette is used to pipette 1150 μl of the DNA polymerase mixed solution for adding to a No. 5 well and pipette 1150 μl of the dNTPs mixed solution (V3.0) for adding to the No. 5 well, and the pipette is used to blow and mix uniformly for 10-15 times. Secondly, a No. 6 well reagent is prepared, namely 1 ml of the pipette is used to pipette 890 μl of the DNA polymerase mixed solution for adding to a No. 6 well and pipette 890 μl of the dNTPs mixed solution II (V2.0) for adding to the No. 6 well, and the pipette is used to blow and mix uniformly for 10-15 times. Then, a No. 14 well reagent is prepared, namely 5 ml of a pipette is used to pipette all No. 14 well reagents, 2.8 ml of the No. 14 well reagent is taken and mixed uniformly with 400 μl of the phi29 polymerase mutant, and added to a No. 14 well. The prepared reagent tanks are assembled. Finally, the on-machine test is performed, namely the sequencer is started and washing is performed, the reagent tank is put into a designated position of the sequencer, and actual preloading is performed in an operating order. After the preloading is finished, the chip prepared in the above step (2) is installed, and related sequencing information is written, namely the sequencing is started. After the end, the chip, the reagent tanks and a washing instrument are taken out. One cycle is tested in the embodiment.

4. Data analysis

After the sequencing is finished, an analysis report is downloaded, a previously specified standard is compared, and the performance of the phi29 DNA polymerase mutant is judged.

Each of comparison parameters is explained as follows: Effective Spots Rates (ESR), and basecall information content (Bic) may be used as a DNB ratio of basecalling; fit (crosstalk fit score) reflects a crosstalk situation, after correction, the intensity distribution of each base is more concentrated, a fit value thereof is higher; a Signal to Noise Ratio (SNR) takes the calculation of a single DNB SNR as an example, base A (max intensity) is served as a signal, CGT is a background, and a variance of the CGT intensity is a noise; Rho Intensity (RHO) is an intensity value of corrected intensity removing normalization, and it is intuitively interpreted as an intensity value of the original intensity of dye after correction.

Results are shown in Fig. 3 (Fig. A is comparison of BIC, FIT, ESR parameters of each mutant; Fig. B is comparison of SNR parameters of each mutant; and Fig. C is comparison of RHO parameters of each mutant), it may be seen that synthesized with the analysis of the above various parameter indexes, the best of the tested mutants is 464, and the second is 463.

## Industrial application

On the basis of the disclosure, the mutants with the good effects may be further screened through performing saturation mutations on the amino acids in the mutation sites of the disclosure; or on the basis of the mutants of the disclosure, and other amino acids except the amino acid sites contained in the disclosure are mutated, to obtain a similar effect. The disclosure may also be applied in the technical fields including food detection, virus detection, RNA detection, single-cell sequencing and the like, and it may also be used to develop third and fourth generation sequencer technologies.

## Claims

**1.** A protein, represented by A) or B) below:

the protein represented by the A) is a protein having DNA polymerase activity that is obtained by modifying at least one amino acid residue(s) in the following 6 sites in a phi29 DNA polymerase amino acid sequence: the 97-th, 123-th, 217-th, 224-th, 515-th, and 474-th sites, wherein the rest amino acid residues are not changed; and the protein represented by the B) is a protein having DNA polymerase activity that is derived from the A) by adding a tag sequence to a terminal of the amino acid sequence of the protein represented by the A).

**2.** The protein according to claim 1, wherein:
the protein represented by the A) is a protein having DNA polymerase activity that is obtained by modifying amino acid residues in at least 2 or at least 3 or at least 4 or at least 5 or all of the following 6 sites in the phi29 DNA polymerase amino acid sequence: the 97-th, 123-th, 217-th, 224-th, 515-th, and 474-th sites, wherein the rest amino acid residues are not changed.

**3.** The protein according to claim 1 or 2, wherein:
the protein represented by the A) is a protein having DNA polymerase activity that is obtained by modifying amino acid residues in all of the following 5 sites in the phi29 DNA polymerase amino acid sequence: the 97-th, 123-th, 217-th, 224-th, and 515-th sites, wherein the rest amino acid residues are not changed.

**4.** The protein according to claim 1 or 2, wherein:
the protein represented by the A) is a protein having DNA polymerase activity that is obtained by modifying amino acid residues in all of the following 3 sites in the phi29 DNA polymerase amino acid sequence: the 224-th, 515-th and 474-th sites, wherein the rest amino acid residues are not changed.

**5.** The protein according to claim 1 or 2, wherein:
the protein represented by the A) is a protein having DNA polymerase activity that is obtained by modifying amino acid residues in all of the following 4 sites in the phi29 DNA polymerase amino acid sequence: the 97-th, 123-th, 217-th and 515-th sites, wherein the rest amino acid residues are not changed.

**6.** The protein according to claim 1 or 2, wherein:
the protein represented by the A) is a protein having DNA polymerase activity that is obtained by modifying amino acid residues in the following 4 sites in the phi29 DNA polymerase amino acid sequence: the 97-th, 123-th, 224-th, and 515-th sites, wherein the rest amino acid residues are not changed.

**7.** The protein according to claim 1 or 2, wherein:
the protein represented by the A) is a protein having DNA polymerase activity that is obtained by modifying amino acid residues in the following 2 sites in the phi29 DNA polymerase amino acid sequence: the 224-th and 474-th sites, wherein the rest amino acid residues are not changed.

**8.** The protein according to claim 1 or 2, wherein:
the protein represented by the A) is a protein having DNA polymerase activity that is obtained by modifying amino acid residues in the following 2 sites in the phi29 DNA polymerase amino acid sequence: the 217-th and 224-th sites, wherein the rest amino acid residues are not changed.

**9.** The protein according to any one of claims 1-8, wherein the modification is amino acid substitution.

**10.** The protein according to claim 9, wherein:
the amino acid substitutions in 6 sites of the 97-th, 123-th, 217-th, 224-th, 515-th and 474-th sites are respectively as follows:

a methionine in the 97-th site is substituted with an alanine or a histidine or a lysine or a threonine;
a leucine in the 123-th site is substituted with a lysine or a phenylalanine or an isoleucine or a histidine;
a glycine in the 217-th site is substituted with a glutamic acid;
a tyrosine in the 224-th site is substituted with a lysine;
an isoleucine in the 474-th site is substituted with a lysine; and
a glutamic acid in the 515-th site is substituted with a proline or a glycine.

**11.** The protein according to claim 10, wherein:
the protein represented by the A) is a protein having DNA polymerase activity that is obtained by mutating the amino acid residue from M into K in the 97-th site, from L into H in the 123-th site, from E into P in the 515-th site, from Y into K in the 224-th site, and from G into K in the 217-th site of the phi29 DNA polymerase amino acid sequence, wherein the rest amino acid residues are not changed.

**12.** The protein according to claim 10, wherein:
the protein represented by the A) is a protein that is obtained by mutating the amino acid residue from Y into K in the 224-th site, from I into K in the 474-th site, and from E into P in the 515-th site of the phi29 DNA polymerase amino acid sequence.

**13.** The protein according to claim 10, wherein:
the protein represented by the A) is a protein that is obtained by mutating the amino acid residue from M into T in the 97-th site, from L into H in the 123-th site, and from E into P in the 515-th site of the phi29 DNA polymerase amino acid sequence.

**14.** The protein according to any one of claims 1-13, wherein: the phi29 DNA polymerase amino acid sequence is any one of the followings:

(I) a protein shown in SEQ ID NO: 2 of a sequence listing;

(II) a protein having more than 90% identity with the protein shown in SEQ ID NO: 2 of the sequence listing and derived from a Bacillus subtilis protein; and
(III) a protein having more than 95% identity with the protein shown in SEQ ID NO: 2 of the sequence listing and derived from a Bacillus subtilis protein.

**15.** The protein according to any one of claims 1-14, wherein: the stability of the protein is higher than that of the phi29 DNA polymerase.

**16.** The protein according to claim 15, wherein: the stability is thermal stability.

**17.** A nucleic acid molecule for encoding the protein according to any one of claims 1-16.

**18.** An expression cassette, a recombinant vector, recombinant bacteria or a transgenic cell line comprising the nucleic acid molecule according to claim 17.

**19.** Use of the protein according to any one of claims 1-16 in at least one of the following 1)-11):

1) as a DNA polymerase;
2) catalyzing DNA replication and/or catalyzing DNA amplification;
3) catalyzing rolling circle amplification and/or catalyzing multiple-strand displacement amplification;
4) performing DNA sequencing or RNA sequencing or whole genome sequencing;
5) constructing a RCA library;
6) detecting a genome amplification coverage;
7) preparing a kit for catalyzing the DNA replication and/or catalyzing the DNA amplification;
8) preparing a product for catalyzing the rolling circle amplification and/or catalyzing the multiple-strand displacement amplification;
9) preparing a product for performing the DNA sequencing or the RNA sequencing or the whole genome sequencing;
10) preparing a product for constructing the RCA library; and
11) preparing a product for detecting the genome amplification coverage.

**20.** Use of the nucleic acid molecule for encoding the protein according to any one of claims 1-16, the expression cassette comprising the nucleic acid molecule, the recombinant vector comprising the nucleic acid molecule, the recombinant bacteria comprising the nucleic acid molecule, or the transgenic cell comprising the nucleic acid molecule in at least one of the following 1)-10):

1) catalyzing DNA replication and/or catalyzing DNA amplification;
2) catalyzing rolling circle amplification and/or catalyzing multiple-strand displacement amplification;
3) performing DNA sequencing or RNA sequencing or whole genome sequencing;
4) constructing a RCA library;
5) detecting a genome amplification coverage;
6) preparing a product for catalyzing the DNA replication and/or catalyzing the DNA amplification;
7) preparing a product for catalyzing the rolling circle amplification and/or catalyzing the multiple-strand displacement amplification;
8) preparing a product for performing the DNA sequencing or the RNA sequencing or the whole genome sequencing;
9) preparing a product for constructing the RCA library; and
10) preparing a product for detecting the genome amplification coverage.

**21.** A method for improving stability of a phi29 DNA polymerase, comprising the following steps: modifying at least one amino acid residue(s) in the following 6 sites of the phi29 DNA polymerase amino acid sequence: the 97-th, 123-th, 217-th, 224-th, 515-th and 474-th sites, in the modification modes according to any one of claims 1-17, wherein the rest amino acid residues are not changed, to obtain a protein having DNA polymerase activity.

**22.** The method according to claim 22, wherein: the stability is thermal stability.

Fig. 1

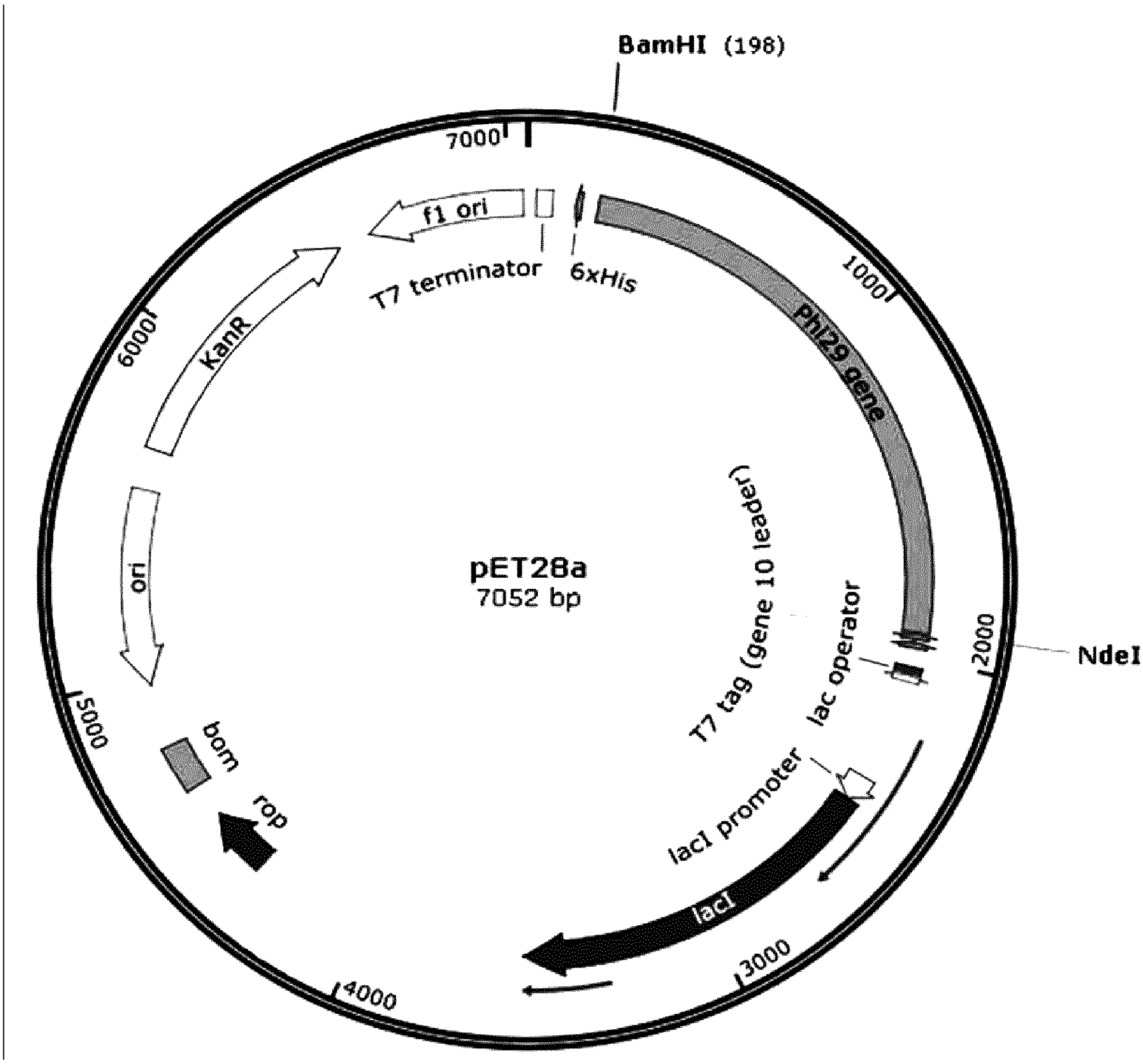
BamHI (198)
7000
f1 ori
T7 terminator
6xHis
1000
Phi29 gene
6000
KanR
ori
pET28a
7052 bp
T7 tag (gene 10 leader)
lac operator
2000
NdeI
5000
bom
rop
lacI promoter
lacI
3000
4000

Fig. 2

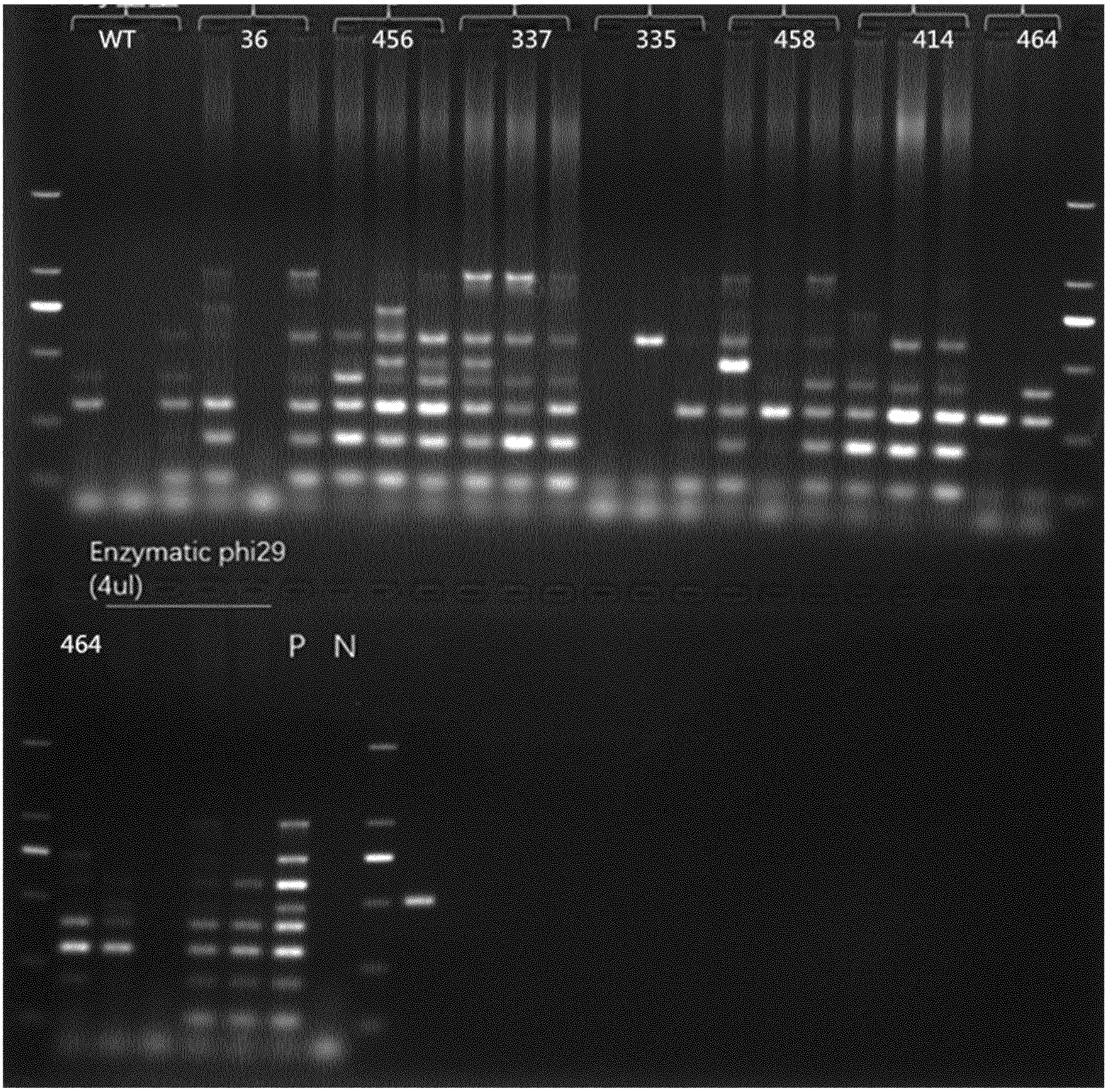

Fig. 3

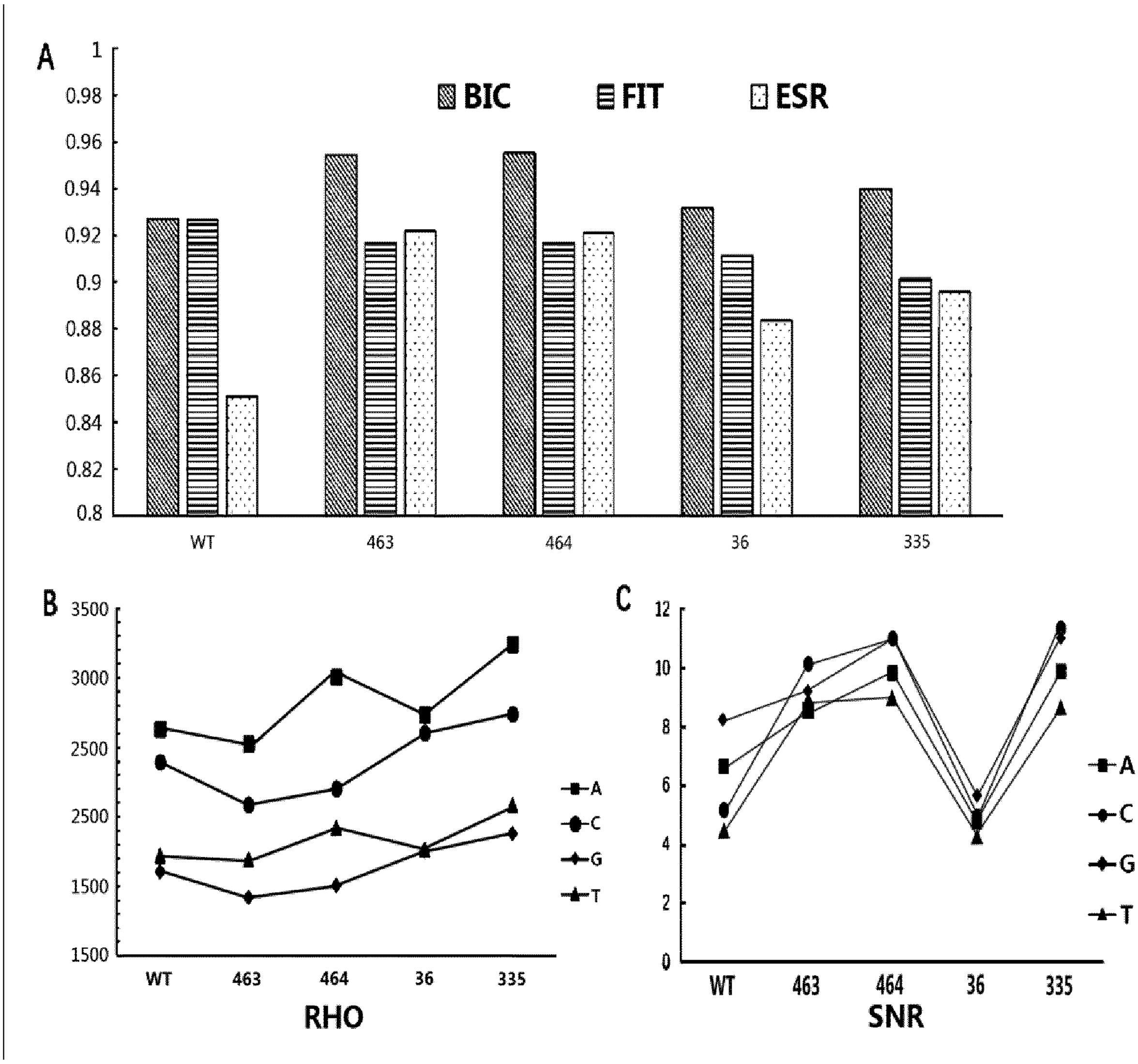
A
BIC
FIT
ESR
1
0.98
0.96
0.94
0.92
0.9
0.88
0.86
0.84
0.82
0.8
WT
463
464
36
335
B
3500
3000
2500
2500
1500
1500
A
C
G
T
RHO
C
12
10
8
6
4
2
0
A
C
G
T
SNR

**INTERNATIONAL SEARCH REPORT**

International application No. **PCT/CN2018/109777**

**A. CLASSIFICATION OF SUBJECT MATTER**

C12N 9/12(2006.01)i；C12N 15/52(2006.01)i；C12N 15/63(2006.01)i

According to International Patent Classification (IPC) or to both national classification and IPC

**B. FIELDS SEARCHED**

Minimum documentation searched (classification system followed by classification symbols)

C12N

Documentation searched other than minimum documentation to the extent that such documents are included in the fields searched

Electronic data base consulted during the international search (name of data base and, where practicable, search terms used)

CPRSABS, CNABS, DWPI, SIPOABS, CNKI, 百度学术, Baidu Scholar, ISI Web of Science, 中国专利生物序列检索系统, China Patent Biological Sequence Search System, Genbank, EMBL, DDBJ: phi29 DNA聚合酶, search for SEQ ID NOs: 1-2, phi29 DNA polymerase

**C. DOCUMENTS CONSIDERED TO BE RELEVANT**

| Category* | Citation of document, with indication, where appropriate, of the relevant passages | Relevant to claim No. |
|---|---|---|
| X | US 9422535 B2 (THERMO FISHER SCIENTIFIC BALTICS UAB) 23 August 2016 (2016-08-23)<br>see abstract, and claims 1-20 | 1, 9-10 and 14-22 (partial technical solution of each claim) |
| A | US 2010112645 A1 (PACIFIC BIOSCIENCES OF CALIFORNIA, INC.) 06 May 2010 (2010-05-06)<br>see entire document | 1, 9-10 and 14-22 (partial technical solution of each claim) |
| A | WO 2011040971 A2 (PACIFIC BIOSCIENCES OF CALIFORNIA, INC.) 07 April 2011 (2011-04-07)<br>see entire document | 1, 9-10 and 14-22 (partial technical solution of each claim) |
| A | US 2011189659 A1 (PACIFIC BIOSCIENCES OF CALIFORNIA, INC.) 04 August 2011 (2011-08-04)<br>see entire document | 1, 9-10 and 14-22 (partial technical solution of each claim) |

☑ Further documents are listed in the continuation of Box C. ☑ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **22 May 2019** | **06 June 2019** |
| Name and mailing address of the ISA/CN<br>**State Intellectual Property Office of the P. R. China**<br>**No. 6, Xitucheng Road, Jimenqiao Haidian District, Beijing 100088**<br>**China** | Authorized officer |
| Facsimile No. **(86-10)62019451** | Telephone No. |

**INTERNATIONAL SEARCH REPORT**

International application No.
**PCT/CN2018/109777**

**C. DOCUMENTS CONSIDERED TO BE RELEVANT**

| Category* | Citation of document, with indication, where appropriate, of the relevant passages | Relevant to claim No. |
|---|---|---|
| A | 练杜鹃, 等 (LIAN, Dujuan et al.). "Phi29 DNA聚合酶最新应用研究进展 (Research Advances in the Latest Application of phi29 DNA Polymerase)" *药物生物技术 (Chinese Journal of Pharmaceutical Biotechnology),* Vol. 23, No. (2), 30 April 2016 (2016-04-30), ISSN: 1005-8915, see pp. 150-154 | 1, 9-10 and 14-22 (partial technical solution of each claim) |
| A | DE VAGA, M. et al. "Mutational Analysis of Ø29 DNA Polymerase Residues Acting as ssDNA Ligands for 3'-5' Exonucleolysis" *Journal of Molecular Biology,* Vol. 279, No. (4), 19 June 1998 (1998-06-19), ISSN: 0022-2836, see pp. 807-822 | 1, 9-10 and 14-22 (partial technical solution of each claim) |

Form PCT/ISA/210 (second sheet) (January 2015)

INTERNATIONAL SEARCH REPORT

International application No. PCT/CN2018/109777

**Box No. III Observations where unity of invention is lacking (Continuation of item 3 of first sheet)**

This International Searching Authority found multiple inventions in this international application, as follows:

[1] Invention 1: claims 1, 9-10 and 14-22 (partial technical solution of each claim),

[2] relating to a protein, which is A) or B) as follows: the protein as shown in A) being a protein having DNA polymerase activity and obtained by modifying an amino acid residue at position 97 in the amino acid sequence of a Phi29 DNA polymerase and remaining the remaining amino acid residues unchanged; and the protein as shown in B) being an A)-derived protein which is obtained by adding a tag sequence to the terminal of the amino acid sequence of the protein as shown in A) and has DNA polymerase activity; a nucleic acid molecule encoding the protein; an expression cassette, a recombinant vector, a recombinant bacteria or a transgenic cell line containing the nucleic acid molecule; an application of the protein; and a method for improving the stability of the Phi29 DNA polymerase by means of amino acid residue modification;

[3] Inventions 2-6: claims 1, 9-10 and 14-22 (partial technical solution of each claim),

[4] relating to a protein, which is A) or B) as follows: the protein as shown in A) being a protein having DNA polymerase activity and obtained by respectively modifying one amino acid residue selected from positions 123, 217, 224, 515 and 474 in the amino acid sequence of a Phi29 DNA polymerase and remaining the remaining amino acid residues unchanged; and the protein as shown in B) being an A)-derived protein which is obtained by adding a tag sequence to the terminal of the amino acid sequence of the protein as shown in A) and has DNA polymerase activity; a nucleic acid molecule encoding the protein; an expression cassette, a recombinant vector, a recombinant bacteria or a transgenic cell line containing the nucleic acid molecule; an application of the protein; and a method for improving the stability of the Phi29 DNA polymerase by means of amino acid residue modification;

[5] Inventions 7-21: claims 1-2, 7-10 and 14-22 (partial technical solution of each claim),

[6] relating to a protein, which is A) or B) as follows: the protein as shown in A) being a protein having DNA polymerase activity and obtained by respectively modifying two amino acid residues selected from positions 97, 123, 217, 224, 515 and 474 in the amino acid sequence of a Phi29 DNA polymerase and remaining the remaining amino acid residues unchanged; and the protein as shown in B) being an A)-derived protein which is obtained by adding a tag sequence to the terminal of the amino acid sequence of the protein as shown in A) and has DNA polymerase activity; a nucleic acid molecule encoding the protein; an expression cassette, a recombinant vector, a recombinant bacteria or a transgenic cell line containing the nucleic acid molecule; an application of the protein; and a method for improving the stability of the Phi29 DNA polymerase by means of amino acid residue modification;

[7] Inventions 22-41: claims 1-2, 4, 9-10 and 12-22 (partial technical solution of each claim),

[8] relating to a protein, which is A) or B) as follows: the protein as shown in A) being a protein having DNA polymerase activity and obtained by respectively modifying three amino acid residues selected from positions 97, 123, 217, 224, 515 and 474 in the amino acid sequence of a Phi29 DNA polymerase and remaining the remaining amino acid residues unchanged; and the protein as shown in B) being an A)-derived protein which is obtained by adding a tag sequence to the terminal of the amino acid sequence of the protein as shown in A) and has DNA polymerase activity; a nucleic acid molecule encoding the protein; an expression cassette, a recombinant vector, a recombinant bacteria or a transgenic cell line containing the nucleic acid molecule; an application of the protein; and a method for improving the stability of the Phi29 DNA polymerase by means of amino acid residue modification;

[9] Inventions 42-56: claims 1-2, 5-6, 9-10 and 14-22 (partial technical solution of each claim),

[10] relating to a protein, which is A) or B) as follows: the protein as shown in A) being the protein having DNA polymerase activity and obtained by respectively modifying four amino acid residues selected from positions 97, 123, 217, 224, 515 and 474 in the amino acid sequence of a Phi29 DNA polymerase and remaining the remaining amino acid residues unchanged; and the protein as shown in B) being an A)-derived protein which is obtained by adding a tag sequence to the terminal of the amino acid sequence of the protein as shown in A) and has DNA polymerase activity; a nucleic acid molecule encoding the protein; an expression cassette, a recombinant vector, a recombinant bacteria or a transgenic cell line containing the nucleic acid molecule; an application of the protein; and a method for improving the stability of the Phi29 DNA polymerase by means of amino acid residue modification;

[11] Inventions 57-62: claims 1-3, 9-11 and 14-22 (partial technical solution of each claim),

**INTERNATIONAL SEARCH REPORT**

International application No.
**PCT/CN2018/109777**

**Box No. III Observations where unity of invention is lacking (Continuation of item 3 of first sheet)**

[12] relating to a protein, which is A) or B) as follows: a protein as shown in A) being the protein having DNA polymerase activity and obtained by respectively modifying five amino acid residues selected from positions 97, 123, 217, 224, 515 and 474 in the amino acid sequence of a Phi29 DNA polymerase and remaining the remaining amino acid residues unchanged; and the protein as shown in B) being an A)-derived protein which is obtained by adding a tag sequence to the terminal of the amino acid sequence of the protein as shown in A) and has DNA polymerase activity; a nucleic acid molecule encoding the protein; an expression cassette, a recombinant vector, a recombinant bacteria or a transgenic cell line containing the nucleic acid molecule; an application of the protein; and a method for improving the stability of the Phi29 DNA polymerase by means of amino acid residue modification; and

[13] Invention 63: claims 1-2, 9-10 and 14-22 (partial technical solution of each claim),

[14] relating to a protein, which is A) or B) as follows: the protein as shown in A) being a protein having DNA polymerase activity and obtained by respectively modifying six amino acid residues selected from positions 97, 123, 217, 224, 515 and 474 in the amino acid sequence of a Phi29 DNA polymerase and remaining the remaining amino acid residues unchanged; and the protein as shown in B) being an A)-derived protein which is obtained by adding a tag sequence to the terminal of the amino acid sequence of the protein as shown in A) and has DNA polymerase activity; a nucleic acid molecule encoding the protein; an expression cassette, a recombinant vector, a recombinant bacteria or a transgenic cell line containing the nucleic acid molecule; an application of the protein; and a method for improving the stability of the Phi29 DNA polymerase by means of amino acid residue modification.

[15] The amino acid sequences of the proteins of Inventions 1-63 are completely different, and also do not share a same structural unit. That is to say, Inventions 1-63 do not share the same or corresponding special technical feature therebetween; and these inventions are not so linked as to form a single general inventive concept. Therefore, these inventions do not comply with PCT Rule 13.1, 13.2 and 13.3.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1, 9-10 and 14-22 (partial technical solution of each claim)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2018/109777**

| Patent document cited in search report | Publication date (day/month/year) | Patent family member(s) | Publication date (day/month/year) |
|---|---|---|---|
| US 9422535 B2 | 23 August 2016 | US 2014322759 A1 | 30 October 2014 |
| | | EP 2813576 A2 | 17 December 2014 |
| | | EP 2813576 A3 | 18 March 2015 |
| | | EP 2813576 B1 | 15 June 2016 |
| | | EP 3093347 A1 | 16 November 2016 |
| US 2010112645 A1 | 06 May 2010 | EP 2271751 A4 | 14 September 2011 |
| | | US 8257954 B2 | 04 September 2012 |
| | | EP 2942404 A1 | 11 November 2015 |
| | | EP 2942404 B1 | 23 November 2016 |
| WO 2011040971 A2 | 07 April 2011 | WO 2011040971 A9 | 04 August 2011 |
| | | WO 2011040971 A3 | 03 November 2011 |
| US 2011189659 A1 | 04 August 2011 | US 8420366 B2 | 16 April 2013 |

Form PCT/ISA/210 (patent family annex) (January 2015)

SEQUENCE LISTING

<110> BGI SHENZHEN

<120> Phi29 DNA polymerase mutant with improved thermal stability and use thereof in sequencing

<160> 2

<170> PatentIn version 3.5

<210> 1
<211> 1728
<212> DNA
<213> Artificial Sequence

<400> 1
```
atgaaacata tgccgcgcaa aatgtatagc tgcgactttg aaaccaccac caaagtggaa        60

gattgccgcg tttgggcgta tggctatatg aacatcgaag accacagcga atacaaaatt       120

ggcaacagcc tggatgaatt tatggcgtgg gtgctgaaag ttcaggcgga tctgtatttt       180

cacaacctga aatttgacgg cgcgttcatt attaactggc tggaacgcaa cggctttaaa       240

tggagcgcgg atggcttacc gaacacctat aacaccatta ttagccgcat gggccagtgg       300

tatatgattg atatctgcct gggctataaa ggcaaacgca agattcatac cgtgatctat       360

gatagcctga agaaactgcc gtttccggtg aaaaaaatcg cgaaggactt taaactgacc       420

gtgctgaaag gcgatattga ctaccataaa gaacgcccgg tgggctataa aattaccccg       480

gaggaatatg cgtacatcaa gaacgacatc cagattattg cggaagcgct gctgattcag       540

tttaaacagg gcctggatcg tatgaccgcg ggtagcgata gcctgaaagg ctttaaggac       600

attatcacca ccaagaagtt caagaaagtg tttccgaccc tgagcctggg cctggataaa       660

gaagtgcgct atgcgtatcg cggtggcttt acctggctga acgatcgctt taaggaaaag       720

gaaattggcg aaggcatggt gtttgatgtg aacagcctgt atccggcgca gatgtatagc       780

cgcctgctgc cgtatggtga accgattgtg tttgaaggca agtatgtgtg ggatgaagat       840

tatccgctgc acattcagca tattcgctgc gaattcgaac tgaaggaagg ctatattccg       900

accattcaga ttaaacgcag ccgcttttat aaaggcaacg agtacctgaa aagcagcggc       960

ggcgaaattg cggatctgtg gctgagcaac gtggatctgg aactgatgaa agaacactac      1020

gatctgtaca acgtggaata tatcagcggc ctgaaattta aagcgaccac cggcctgttt      1080

aaggacttta tcgacaagtg gacctacatt aaaaccacca gcgaaggcgc gattaaacag      1140

ctggcgaaac tgatgctgaa cagcctgtat ggcaaatttg cgagcaaccc ggatgttacc      1200

ggcaaagtgc cgtatctgaa agaaaacggc gcgctgggct ttcgtttagg cgaagaggaa      1260

accaaagatc cggtgtatac cccgatgggc gtgtttatta ccgcgtgggc gcgctatacc      1320

accattaccg cggcgcaggc gtgttatgat cgcattatct attgcgatac cgatagcatt      1380
```

```
catctgaccg gcaccgaaat tccggatgtg atcaaagata ttgtggaccc gaaaaaactg     1440

ggctattggg cgcatgaaag cacctttaaa cgcgcgaaat atctgcgcca gaaaacctat     1500

atccaggaca tctacatgaa agaggtggat ggcaaactgg ttgaaggcag cccggatgat     1560

tataccgata ttaagttcag cgtgaaatgc gcgggcatga ccgataaaat taagaaggaa     1620

gtgaccttcg agaactttaa agtgggcttt agccgcaaaa tgaaaccgaa accggttcag     1680

gtgcctggcg gtgttgttct ggtggatgat accttcacca tcaagtga                  1728


<210>  2
<211>  575
<212>  PRT
<213>  Artificial Sequence


<400>  2

Met Lys His Met Pro Arg Lys Met Tyr Ser Cys Asp Phe Glu Thr Thr
1               5                   10                  15


Thr Lys Val Glu Asp Cys Arg Val Trp Ala Tyr Gly Tyr Met Asn Ile
            20                  25                  30


Glu Asp His Ser Glu Tyr Lys Ile Gly Asn Ser Leu Asp Glu Phe Met
        35                  40                  45


Ala Trp Val Leu Lys Val Gln Ala Asp Leu Tyr Phe His Asn Leu Lys
    50                  55                  60


Phe Asp Gly Ala Phe Ile Ile Asn Trp Leu Glu Arg Asn Gly Phe Lys
65                  70                  75                  80


Trp Ser Ala Asp Gly Leu Pro Asn Thr Tyr Asn Thr Ile Ile Ser Arg
                85                  90                  95


Met Gly Gln Trp Tyr Met Ile Asp Ile Cys Leu Gly Tyr Lys Gly Lys
            100                 105                 110


Arg Lys Ile His Thr Val Ile Tyr Asp Ser Leu Lys Lys Leu Pro Phe
        115                 120                 125


Pro Val Lys Lys Ile Ala Lys Asp Phe Lys Leu Thr Val Leu Lys Gly
    130                 135                 140


Asp Ile Asp Tyr His Lys Glu Arg Pro Val Gly Tyr Lys Ile Thr Pro
145                 150                 155                 160


Glu Glu Tyr Ala Tyr Ile Lys Asn Asp Ile Gln Ile Ile Ala Glu Ala
```

```
                165                 170                 175

Leu Leu Ile Gln Phe Lys Gln Gly Leu Asp Arg Met Thr Ala Gly Ser
            180                 185                 190

Asp Ser Leu Lys Gly Phe Lys Asp Ile Ile Thr Thr Lys Lys Phe Lys
        195                 200                 205

Lys Val Phe Pro Thr Leu Ser Leu Gly Leu Asp Lys Glu Val Arg Tyr
    210                 215                 220

Ala Tyr Arg Gly Gly Phe Thr Trp Leu Asn Asp Arg Phe Lys Glu Lys
225                 230                 235                 240

Glu Ile Gly Glu Gly Met Val Phe Asp Val Asn Ser Leu Tyr Pro Ala
                245                 250                 255

Gln Met Tyr Ser Arg Leu Leu Pro Tyr Gly Glu Pro Ile Val Phe Glu
            260                 265                 270

Gly Lys Tyr Val Trp Asp Glu Asp Tyr Pro Leu His Ile Gln His Ile
        275                 280                 285

Arg Cys Glu Phe Glu Leu Lys Glu Gly Tyr Ile Pro Thr Ile Gln Ile
    290                 295                 300

Lys Arg Ser Arg Phe Tyr Lys Gly Asn Glu Tyr Leu Lys Ser Ser Gly
305                 310                 315                 320

Gly Glu Ile Ala Asp Leu Trp Leu Ser Asn Val Asp Leu Glu Leu Met
                325                 330                 335

Lys Glu His Tyr Asp Leu Tyr Asn Val Glu Tyr Ile Ser Gly Leu Lys
            340                 345                 350

Phe Lys Ala Thr Thr Gly Leu Phe Lys Asp Phe Ile Asp Lys Trp Thr
        355                 360                 365

Tyr Ile Lys Thr Thr Ser Glu Gly Ala Ile Lys Gln Leu Ala Lys Leu
    370                 375                 380

Met Leu Asn Ser Leu Tyr Gly Lys Phe Ala Ser Asn Pro Asp Val Thr
385                 390                 395                 400

Gly Lys Val Pro Tyr Leu Lys Glu Asn Gly Ala Leu Gly Phe Arg Leu
                405                 410                 415
```

```
Gly Glu Glu Glu Thr Lys Asp Pro Val Tyr Thr Pro Met Gly Val Phe
            420                 425                 430

Ile Thr Ala Trp Ala Arg Tyr Thr Thr Ile Thr Ala Ala Gln Ala Cys
        435                 440                 445

Tyr Asp Arg Ile Ile Tyr Cys Asp Thr Asp Ser Ile His Leu Thr Gly
    450                 455                 460

Thr Glu Ile Pro Asp Val Ile Lys Asp Ile Val Asp Pro Lys Lys Leu
465                 470                 475                 480

Gly Tyr Trp Ala His Glu Ser Thr Phe Lys Arg Ala Lys Tyr Leu Arg
                485                 490                 495

Gln Lys Thr Tyr Ile Gln Asp Ile Tyr Met Lys Glu Val Asp Gly Lys
            500                 505                 510

Leu Val Glu Gly Ser Pro Asp Asp Tyr Thr Asp Ile Lys Phe Ser Val
        515                 520                 525

Lys Cys Ala Gly Met Thr Asp Lys Ile Lys Lys Glu Val Thr Phe Glu
    530                 535                 540

Asn Phe Lys Val Gly Phe Ser Arg Lys Met Lys Pro Lys Pro Val Gln
545                 550                 555                 560

Val Pro Gly Gly Val Val Leu Val Asp Asp Thr Phe Thr Ile Lys
                565                 570                 575
```